# EUROPEAN PATENT APPLICATION

(11) **EP 4 803 547 A1**
(43) Date of publication of application: **09.09.2026**
(21) Application number: 24885028.1
(22) Date of filing: 04.11.2024
(51) Int. Cl.: C07K 16/46, C07K 16/30, C12N 15/13, C12N 15/63, A61K 39/395, A61P 35/00

(54) **ANTI-PD-1/CD40 BISPECIFIC ANTIBODY AND USE THEREOF**

(30) Priority: 03.11.2023 CN 202311466590
(71) Applicant: Biontech (Zhuhai) Pharmaceuticals R&D Co., Ltd, Zhuhai, Guangdong 519080 (CN)
(72) Inventor: JIA, Yunli, Zhuhai, Guangdong 519080 (CN); ZHANG, Zhenqing, Zhuhai, Guangdong 519080 (CN); HUANG, Weifeng, Zhuhai, Guangdong 519080 (CN); TSUN, Andy, Zhuhai, Guangdong 519080 (CN)
(74) Representative: Maiwald GmbH
(86) International application number: PCT/CN2024/129694
(87) International publication number: WO 2025/093033

(57) **Abstract**

The present invention relates to a bispecific antibody or an antigen-binding fragment thereof capable of specifically binding to CD40 and PD-1, a pharmaceutical composition comprsing same, and use thereof.

## Description

This application is based upon and claims priority to CN Application No. 202311466590.2, filed on November 03, 2023, the disclosure of which is incorporated herein by reference in its entirety.

### TECHNICAL FIELD

The present application belongs to the field of biomedical technology. More specifically, the present application relates to a bispecific antibody or antigen-binding fragment thereof capable of specifically binding to CD40 and PD-1, as well as a pharmaceutical composition comprising the same and use thereof.

### BACKGROUND

CD40, also known as TNFRSF5, is a member of the tumor necrosis factor receptor (TNFR) superfamily. It is expressed on antigen-presenting cells (APCs), including dendritic cells (DCs), B cells, macrophages, classical and non-classical monocytes, as well as a variety of non-immune cells, including platelets and endothelial cells, and several types of tumor cells. The CD40 precursor contains 297 aa and is a type I transmembrane glycoprotein composed of an N-terminal signal peptide (20 aa), an extracellular domain (193 aa), a transmembrane domain (22 aa), and a cytoplasmic domain (62 aa). The extracellular domain contains four CDRs with a total of 22 cysteine residues. CD40 is highly glycosylated; its molecular weight, calculated from its amino acid sequence, is 28 kD, but the glycosylated CD40 is 40-50 kD.

The cognate ligand for CD40 is CD154 (TNFSF5/CD40L), a type II transmembrane protein with a molecular weight of approximately 39 kDa. The expression of CD40L is generally inducible and restricted to cells of the hematopoietic system, such as platelets, granulocytes, activated T cells, activated B cells, and activated natural killer (NK) cells, although weak expression is also observed in endothelial cells and smooth muscle cells. Numerous studies have confirmed that CD40-CD40L plays a crucial role in the function of CD8 cytotoxic T lymphocytes (CTLs) in the immune response and is essential for adaptive immune responses.

The widespread expression of CD40 on monocytes and their mature progeny, DCs and macrophages, as well as B cells, plays a crucial role in the function of immune cells. Monocytes are progenitor cells of innate immunity with high plasticity. They have the ability to differentiate into various cell types, such as myeloid-derived suppressor cells (MDSCs), macrophages, and DCs. CD40 signaling is an important trigger in the maturation process of monocytes, primarily driving differentiation into macrophages of the M1 lineage and DCs. The binding of CD40 to the surface of DCs promotes the production of cytokines and chemokines, induces the expression of co-stimulatory molecules, and facilitates the cross-presentation of antigens. One of the primary functions of CD40L is to enhance antigen-presenting activity by activating DCs. This step, known as "licensing," increases the interaction between DCs and T cells by upregulating surface proteins such as CD54 and CD86, thereby activating the latter. However, most CD40 agonists have a narrow therapeutic window, posing certain challenges to their development.

Programmed death 1 (PD-1, also known as CD279) is typically expressed on tumor-infiltrating lymphocytes. It can inhibit the activation and proliferation of T cells by binding to programmed death ligand 1 expressed on tumor cells and antigen-presenting cells, thereby exerting a negative immunoregulatory effect and mediating tumor immune evasion. The PD-1 pathway is a cornerstone of immunomodulation. Inhibitory antibodies targeting PD-1 or PD-L1 (nivolumab, pembrolizumab, cemiplimab; atezolizumab, avelumab, durvalumab) have been approved by the U.S. Food and Drug Administration (FDA) for the treatment of various cancers. Despite these clinical successes, the majority of patients do not exhibit a complete response, and many experience immune-related adverse events, highlighting the need for a better understanding of how to safely and effectively modulate the PD-1 pathway in patients.

### SUMMARY OF THE INVENTION

Based on extensive research, the inventors of the present application have developed a bispecific antibody capable of specifically binding to PD-1 and CD40. The bispecific antibody provided herein is capable of: blocking the PD-1/PD-L1 signaling pathway, activating the CD40 downstream signaling pathway (e.g., the NF-κB pathway) in a manner dependent on binding to PD-1, activating primary B cells, activating T cells in a mixed lymphocyte reaction system, and/or inhibiting tumor growth in vivo.

Furthermore, the bispecific antibody provided herein also possesses cross-binding activity with human CD40 and monkey CD40, as well as cross-binding activity with human PD-1 and monkey PD-1.

In certain embodiments, the bispecific antibody provided herein is capable of blocking both the binding of PD-1 to PD-L1 and the binding of CD40 to CD40L. In certain embodiments, the bispecific antibody provided herein is capable of blocking the binding of PD-1 to PD-L1 but does not block the binding of CD40 to CD40L.

In certain embodiments, the bispecific antibody provided herein exhibits significant anti-tumor activity while avoiding systemic immune activation effects, thereby reducing toxic side effects.

Therefore, in one aspect, the present application provides a bispecific antibody comprising a first antigen-binding domain that specifically binds to PD-1 and a second antigen-binding domain that specifically binds to CD40;
wherein the first antigen-binding domain comprises a first light chain variable region (VL) and a first heavy chain variable region (VH) which together form a domain capable of specifically binding to PD-1; and the second antigen-binding domain comprises a second light chain variable region (VL) and a second heavy chain variable region (VH) which together form a domain capable of specifically binding to CD40.

In certain embodiments, the first antigen-binding domain and the second antigen-binding domain are each independently an scFv or a Fab.

In certain embodiments, the first light chain variable region (VL) comprises a LCDR1, a LCDR2, and a LCDR3 comprised in the VL as set forth in SEQ ID NO: 4; and/or the first heavy chain variable region (VH) comprises a HCDR1, a HCDR2, and a HCDR3 comprised in the VH as set forth in SEQ ID NO: 3.

In certain embodiments, the first light chain variable region (VL) comprises a LCDR1, a LCDR2, and a LCDR3 comprised in the VL as set forth in SEQ ID NO: 40; and/or the first heavy chain variable region (VH) comprises a HCDR1, a HCDR2, and a HCDR3 comprised in the VH as set forth in SEQ ID NO: 39.

In certain embodiments, the CDRs are defined by the Kabat, Chothia, Abm, or IMGT numbering system.

In certain embodiments, the first light chain variable region (VL) comprises a LCDR1 as set forth in SEQ ID NO: 16, a LCDR2 as set forth in SEQ ID NO: 17, and a LCDR3 as set forth in SEQ ID NO: 18; and/or the first heavy chain variable region (VH) comprises a HCDR1 as set forth in SEQ ID NO: 13, a HCDR2 as set forth in SEQ ID NO: 14, and a HCDR3 as set forth in SEQ ID NO: 15.

In certain embodiments, the first light chain variable region (VL) comprises a LCDR1 as set forth in SEQ ID NO: 44, a LCDR2 as set forth in SEQ ID NO: 45, and a LCDR3 as set forth in SEQ ID NO: 46; and/or the first heavy chain variable region (VH) comprises a HCDR1 as set forth in SEQ ID NO: 41, a HCDR2 as set forth in SEQ ID NO: 42, and a HCDR3 as set forth in SEQ ID NO: 43.

In certain embodiments, the CDRs are defined by the IMGT numbering system.

In certain embodiments of the bispecific antibody of the present application, (a) the first light chain variable region (VL) comprises an amino acid sequence as set forth in SEQ ID NO: 4 or a variant thereof, and/or the first heavy chain variable region (VH) comprises an amino acid sequence as set forth in SEQ ID NO: 3 or a variant thereof; or (b) the first light chain variable region (VL) comprises an amino acid sequence as set forth in SEQ ID NO: 40 or a variant thereof, and/or the first heavy chain variable region (VH) comprises an amino acid sequence as set forth in SEQ ID NO: 39 or a variant thereof;
wherein the variant has one or several amino acid substitutions, deletions, or additions (e.g., 1, 2, 3, 4, or 5 amino acid substitutions, deletions, or additions) compared to the sequence from which it is derived, or has a sequence having at least 80%, at least 85%, at least 90%, at least 91%, at least 92%, at least 93%, at least 94%, at least 95%, at least 96%, at least 97%, at least 98%, at least 99%, or 100% sequence identity thereto; preferably, the substitutions are conservative substitutions.

In certain embodiments, the second light chain variable region (VL) comprises a LCDR1, a LCDR2, and a LCDR3 comprised in the VL as set forth in SEQ ID NO: 23 or 33; and/or the second heavy chain variable region (VH) comprises a HCDR1, a HCDR2, and a HCDR3 comprised in the VH as set forth in SEQ ID NO: 5 or 31.

In certain embodiments, the second light chain variable region (VL) comprises a LCDR1, a LCDR2, and a LCDR3 comprised in the VL as set forth in SEQ ID NO: 24 or 34; and/or the second heavy chain variable region (VH) comprises a HCDR1, a HCDR2, and a HCDR3 comprised in the VH as set forth in SEQ ID NO: 6 or 32.

In certain embodiments, the CDRs are defined by the Kabat, Chothia, Abm, or IMGT numbering system.

In certain embodiments, the second light chain variable region (VL) comprises a LCDR1 as set forth in SEQ ID NO: 25, a LCDR2 as set forth in SEQ ID NO: 26, and a LCDR3 as set forth in SEQ ID NO: 27; and/or the second heavy chain variable region (VH) comprises a HCDR1 as set forth in SEQ ID NO: 7, a HCDR2 as set forth in SEQ ID NO: 8, and a HCDR3 as set forth in SEQ ID NO: 9.

In certain embodiments, the second light chain variable region (VL) comprises a LCDR1 as set forth in SEQ ID NO: 28, a LCDR2 as set forth in SEQ ID NO: 29, and a LCDR3 as set forth in SEQ ID NO: 30; and/or the second heavy chain variable region (VH) comprises a HCDR1 as set forth in SEQ ID NO: 10, a HCDR2 as set forth in SEQ ID NO: 11, and a HCDR3 as set forth in SEQ ID NO: 12.

In certain embodiments, the CDRs are defined by the IMGT numbering system.

In certain embodiments of the bispecific antibody of the present application, (a) the second light chain variable region (VL) comprises an amino acid sequence as set forth in SEQ ID NO: 23 or 33 or a variant thereof, and/or the second heavy chain variable region (VH) comprises an amino acid sequence as set forth in SEQ ID NO: 5 or 31 or a variant thereof; or (b) the second light chain variable region (VL) comprises an amino acid sequence as set forth in SEQ ID NO: 24 or 34 or a variant thereof, and/or the second heavy chain variable region (VH) comprises an amino acid sequence as set forth in SEQ ID NO: 6 or 32 or a variant thereof;
wherein the variant has one or several amino acid substitutions, deletions, or additions (e.g., 1, 2, 3, 4, or 5 amino acid substitutions, deletions, or additions) compared to the sequence from which it is derived, or has a sequence having at least 80%, at least 85%, at least 90%, at least 91%, at least 92%, at least 93%, at least 94%, at least 95%, at least 96%, at least 97%, at least 98%, at least 99%, or 100% sequence identity thereto; preferably, the substitutions are conservative substitutions.

In certain embodiments, the second light chain variable region (VL) comprises an amino acid sequence as set forth in SEQ ID NO: 23, and/or the second heavy chain variable region (VH) comprises an amino acid sequence as set forth in SEQ ID NO: 5.

In certain embodiments, the second light chain variable region (VL) comprises an amino acid sequence as set forth in SEQ ID NO: 33, and/or the second heavy chain variable region (VH) comprises an amino acid sequence as set forth in SEQ ID NO: 31.

In certain embodiments, the second light chain variable region (VL) comprises an amino acid sequence as set forth in SEQ ID NO: 24, and/or the second heavy chain variable region (VH) comprises an amino acid sequence as set forth in SEQ ID NO: 6.

In certain embodiments, the second light chain variable region (VL) comprises an amino acid sequence as set forth in SEQ ID NO: 34, and/or the second heavy chain variable region (VH) comprises an amino acid sequence as set forth in SEQ ID NO: 32.

In certain embodiments, the bispecific antibody comprises:
(1) the first light chain variable region (VL) comprising a LCDR1, a LCDR2, and a LCDR3 comprised in the VL as set forth in SEQ ID NO: 4, the first heavy chain variable region (VH) comprising a HCDR1, a HCDR2, and a HCDR3 comprised in the VH as set forth in SEQ ID NO: 3, the second light chain variable region (VL) comprising a LCDR1, a LCDR2, and a LCDR3 comprised in the VL as set forth in SEQ ID NO: 23 or 33, and the second heavy chain variable region (VH) comprising a HCDR1, a HCDR2, and a HCDR3 comprised in the VH as set forth in SEQ ID NO: 5 or 31;
(2) the first light chain variable region (VL) comprising a LCDR1, a LCDR2, and a LCDR3 comprised in the VL as set forth in SEQ ID NO: 4, the first heavy chain variable region (VH) comprising a HCDR1, a HCDR2, and a HCDR3 comprised in the VH as set forth in SEQ ID NO: 3, the second light chain variable region (VL) comprising a LCDR1, a LCDR2, and a LCDR3 comprised in the VL as set forth in SEQ ID NO: 24 or 34, and the second heavy chain variable region (VH) comprising a HCDR1, a HCDR2, and a HCDR3 comprised in the VH as set forth in SEQ ID NO: 6 or 32;
(3) the first light chain variable region (VL) comprising a LCDR1, a LCDR2, and a LCDR3 comprised in the VL as set forth in SEQ ID NO: 40, the first heavy chain variable region (VH) comprising a HCDR1, a HCDR2, and a HCDR3 comprised in the VH as set forth in SEQ ID NO: 39, the second light chain variable region (VL) comprising a LCDR1, a LCDR2, and a LCDR3 comprised in the VL as set forth in SEQ ID NO: 23 or 33, and the second heavy chain variable region (VH) comprising a HCDR1, a HCDR2, and a HCDR3 comprised in the VH as set forth in SEQ ID NO: 5 or 31; or
(4) the first light chain variable region (VL) comprising a LCDR1, a LCDR2, and a LCDR3 comprised in the VL as set forth in SEQ ID NO: 40, the first heavy chain variable region (VH) comprising a HCDR1, a HCDR2, and a HCDR3 comprised in the VH as set forth in SEQ ID NO: 39, the second light chain variable region (VL) comprising a LCDR1, a LCDR2, and a LCDR3 comprised in the VL as set forth in SEQ ID NO: 24 or 34, and the second heavy chain variable region (VH) comprising a HCDR1, a HCDR2, and a HCDR3 comprised in the VH as set forth in SEQ ID NO: 6 or 32.

In certain embodiments, the bispecific antibody comprises:
(1) the first light chain variable region (VL) comprising a LCDR1 as set forth in SEQ ID NO: 16, a LCDR2 as set forth in SEQ ID NO: 17, and a LCDR3 as set forth in SEQ ID NO: 18, the first heavy chain variable region (VH) comprising a HCDR1 as set forth in SEQ ID NO: 13, a HCDR2 as set forth in SEQ ID NO: 14, and a HCDR3 as set forth in SEQ ID NO: 15; the second light chain variable region (VL) comprising a LCDR1 as set forth in SEQ ID NO: 25, a LCDR2 as set forth in SEQ ID NO: 26, and a LCDR3 as set forth in SEQ ID NO: 27, and the second heavy chain variable region (VH) comprising a HCDR1 as set forth in SEQ ID NO: 7, a HCDR2 as set forth in SEQ ID NO: 8, and a HCDR3 as set forth in SEQ ID NO: 9;
(2) the first light chain variable region (VL) comprising a LCDR1 as set forth in SEQ ID NO: 16, a LCDR2 as set forth in SEQ ID NO: 17, and a LCDR3 as set forth in SEQ ID NO: 18, the first heavy chain variable region (VH) comprising a HCDR1 as set forth in SEQ ID NO: 13, a HCDR2 as set forth in SEQ ID NO: 14, and a HCDR3 as set forth in SEQ ID NO: 15; the second light chain variable region (VL) comprising a LCDR1 as set forth in SEQ ID NO: 28, a LCDR2 as set forth in SEQ ID NO: 29, and a LCDR3 as set forth in SEQ ID NO: 30, and the second heavy chain variable region (VH) comprising a HCDR1 comprising the amino acid sequence as set forth in SEQ ID NO: 10, a HCDR2 comprising the amino acid sequence as set forth in SEQ ID NO: 11, and a HCDR3 comprising the amino acid sequence as set forth in SEQ ID NO: 12;
(3) the first light chain variable region (VL) comprising a LCDR1 as set forth in SEQ ID NO: 44, a LCDR2 as set forth in SEQ ID NO: 45, and a LCDR3 as set forth in SEQ ID NO: 46, the first heavy chain variable region (VH) comprising a HCDR1 as set forth in SEQ ID NO: 41, a HCDR2 as set forth in SEQ ID NO: 42, and a HCDR3 as set forth in SEQ ID NO: 43, the second light chain variable region (VL) comprising a LCDR1 as set forth in SEQ ID NO: 25, a LCDR2 as set forth in SEQ ID NO: 26, and a LCDR3 as set forth in SEQ ID NO: 27, and the second heavy chain variable region (VH) comprising a HCDR1 as set forth in SEQ ID NO: 7, a HCDR2 as set forth in SEQ ID NO: 8, and a HCDR3 as set forth in SEQ ID NO: 9; or
(4) the first light chain variable region (VL) comprising a LCDR1 as set forth in SEQ ID NO: 44, a LCDR2 as set forth in SEQ ID NO: 45, and a LCDR3 as set forth in SEQ ID NO: 46, the first heavy chain variable region (VH) comprising a HCDR1 as set forth in SEQ ID NO: 41, a HCDR2 as set forth in SEQ ID NO: 42, and a HCDR3 as set forth in SEQ ID NO: 43, the second light chain variable region (VL) comprising a LCDR1 as set forth in SEQ ID NO: 28, a LCDR2 as set forth in SEQ ID NO: 29, and a LCDR3 as set forth in SEQ ID NO: 30, and the second heavy chain variable region (VH) comprising a HCDR1 comprising the amino acid sequence as set forth in SEQ ID NO: 10, a HCDR2 comprising the amino acid sequence as set forth in SEQ ID NO: 11, and a HCDR3 comprising the amino acid sequence as set forth in SEQ ID NO: 12.

In certain embodiments, the bispecific antibody comprises:
(1) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 23, and the second heavy chain variable region (VH) comprising the amino acid sequence as set forth in SEQ ID NO: 5;
(2) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 33, and the second heavy chain variable region (VH) comprising the amino acid sequence as set forth in SEQ ID NO: 31;
(3) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 24, and the second heavy chain variable region (VH) comprising the amino acid sequence as set forth in SEQ ID NO: 6;
(4) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 34, and the second heavy chain variable region (VH) comprising the amino acid sequence as set forth in SEQ ID NO: 32;
(5) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 23, and the second heavy chain variable region (VH) comprising the amino acid sequence as set forth in SEQ ID NO: 5;
(6) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 33, and the second heavy chain variable region (VH) comprising the amino acid sequence as set forth in SEQ ID NO: 31;
(7) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 24, and the second heavy chain variable region (VH) comprising the amino acid sequence as set forth in SEQ ID NO: 6; or
(8) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 34, and the second heavy chain variable region (VH) comprising the amino acid sequence as set forth in SEQ ID NO: 32.

In certain embodiments, the first antigen-binding domain is a Fab and the second antigen-binding domain is an scFv; or the first antigen-binding domain is an scFv and the second antigen-binding domain is a Fab.
In certain embodiments, the first antigen-binding domain is a Fab and the second antigen-binding domain is an scFv.

In certain embodiments, the bispecific antibody comprises two of the first antigen-binding domains and two of the second antigen-binding domains; wherein the two first antigen-binding domains are identical or different, and the two second antigen-binding domains are identical or different.

In certain embodiments, the bispecific antibody comprises a peptide chain I and a peptide chain II; wherein the peptide chain I comprises the first light chain variable region and a light chain constant region, and the peptide chain II comprises: the first heavy chain variable region, a heavy chain constant region, the second heavy chain variable region, and the second light chain variable region.

In certain embodiments, the peptide chain I comprises, from N-terminus to C-terminus, the first light chain variable region and a light chain constant region, and/or the peptide chain II comprises, from N-terminus to C-terminus: (i) the first heavy chain variable region, a heavy chain constant region, the second heavy chain variable region, and the second light chain variable region; or (ii) the first heavy chain variable region, a heavy chain constant region, the second light chain variable region, and the second heavy chain variable region.

In certain embodiments, adjacent domains of the peptide chain I are optionally connected via or without a linker, and/or adjacent domains of the peptide chain II are optionally connected via or without a linker.

In certain embodiments, the linkers are each independently the same or a different peptide linker (e.g., a rigid peptide linker or a flexible peptide linker); preferably, the peptide linker is each independently selected from a peptide linker comprising one or more glycines (G) and/or serines (S) and/or alanines (A), e.g., having the structure represented by (GGGGS)n or (GGGGA)n, wherein n is selected from 1, 2, 3, 4, 5, 6, 7, 8, 9, or 10; preferably, the peptide linker comprises an amino acid sequence as set forth in SEQ ID NO: 36 or 37.

In certain embodiments, the peptide chain II comprises, from N-terminus to C-terminus, the first heavy chain variable region, a heavy chain constant region, the second heavy chain variable region, and the second light chain variable region, wherein the heavy chain constant region and the second heavy chain variable region are connected via a peptide linker comprising one or more glycines (G) and/or serines (S) (e.g., a peptide linker as set forth in SEQ ID NO: 36); or the peptide chain II comprises, from N-terminus to C-terminus, the first heavy chain variable region, a heavy chain constant region, the second heavy chain variable region, and the second light chain variable region, wherein the heavy chain constant region and the second heavy chain variable region are connected via a peptide linker comprising one or more glycines (G) and/or alanines (A) (e.g., the peptide linker as set forth in SEQ ID NO: 36);
and/or
the second heavy chain variable region and the second light chain variable region are connected via a peptide linker comprising one or more glycines (G) and/or serines (S) (e.g., a peptide linker as set forth in SEQ ID NO: 37).

In certain embodiments, an intrachain disulfide bond can be formed between the second light chain variable region and the second heavy chain variable region.

In certain embodiments, (a) the second light chain variable region comprises the amino acid sequence as set forth in SEQ ID NO: 33, and the second heavy chain variable region comprises the amino acid sequence as set forth in SEQ ID NO: 31; or (b) the second light chain variable region comprises the amino acid sequence as set forth in SEQ ID NO: 34, and the second heavy chain variable region comprises the amino acid sequence as set forth in SEQ ID NO: 32.

In certain embodiments, the heavy chain constant region is derived from a human immunoglobulin (e.g., IgG1, IgG2, IgG3, or IgG4).

In certain embodiments, the CH2 domain (residues 231-340 of human IgG1, numbered according to the Eu numbering system), the CH3 domain (residues 341-447 of human IgG1, numbered according to the Eu numbering system), and/or the hinge region (residues 216-230, numbered according to the Eu numbering system) of the heavy chain constant region have one, two, or more mutations (e.g., amino acid substitutions) introduced to alter one or more functional properties of the bispecific antibody, such as serum half-life, complement binding, Fc receptor binding, and/or antigen-dependent cellular cytotoxicity.

In certain embodiments, the CH2 domain, CH3 domain, and/or hinge region of the heavy chain constant region have one, two, or more mutations (e.g., amino acid substitutions) introduced, e.g., to reduce or ablate the effector function of the Fc region.

In certain embodiments, the heavy chain constant region is selected from a heavy chain constant region of a wild-type human immunoglobulin (e.g., IgG1, IgG2, IgG3, or IgG4) or a variant thereof (e.g., a heavy chain constant region comprising a mutation or a chemical modification); wherein the Fc domain comprised in the heavy chain constant region variant has altered (e.g., enhanced or reduced) effector functions compared to the heavy chain constant region of the wild-type immunoglobulin from which it is derived; e.g., the Fc domain comprised in the heavy chain constant region variant has reduced ADCC, ADCP, and/or CDC activity compared to the heavy chain constant region of the wild-type immunoglobulin from which it is derived.

In certain embodiments, the heavy chain constant region is selected from a heavy chain constant region of a wild-type human immunoglobulin IgG1 or a variant thereof (e.g., a heavy chain constant region comprising a mutation or a chemical modification); wherein the heavy chain constant region variant comprises L234A, L235A, and/or L237A substitution mutations compared to the heavy chain constant region of the wild-type immunoglobulin from which it is derived. In certain embodiments, the positions of the mutations are determined according to the Eu numbering system.

In certain embodiments, the Fc domain monomer comprises an amino acid sequence as set forth in SEQ ID NO: 19.

In certain embodiments, the light chain constant region is derived from a kappa (κ) or lambda (λ) light chain of a human immunoglobulin.

In certain embodiments, the light chain constant region comprises an amino acid sequence as set forth in SEQ ID NO: 20.

In certain embodiments of the bispecific antibody of the present application:
(a) the peptide chain I comprises an amino acid sequence as set forth in SEQ ID NO: 35 or a variant thereof, and/or the peptide chain II comprises an amino acid sequence as set forth in SEQ ID NO: 1 or 2 or a variant thereof; or
(b) the peptide chain I comprises an amino acid sequence as set forth in SEQ ID NO: 47 or a variant thereof, and/or the peptide chain II comprises an amino acid sequence as set forth in SEQ ID NO: 38 or a variant thereof;
wherein the variant has one or several amino acid substitutions, deletions, or additions (e.g., 1, 2, 3, 4, or 5 amino acid substitutions, deletions, or additions) compared to the sequence from which it is derived, or has a sequence having at least 80%, at least 85%, at least 90%, at least 91%, at least 92%, at least 93%, at least 94%, at least 95%, at least 96%, at least 97%, at least 98%, at least 99%, or 100% sequence identity thereto; preferably, the substitutions are conservative substitutions;

In certain embodiments, the bispecific antibody comprises:
(1) the peptide chain I comprising the amino acid sequence as set forth in SEQ ID NO: 35, and the peptide chain II comprising the amino acid sequence as set forth in SEQ ID NO: 1;
(2) the peptide chain I comprising the amino acid sequence as set forth in SEQ ID NO: 35, and the peptide chain II comprising the amino acid sequence as set forth in SEQ ID NO: 2; or
(3) the peptide chain I comprising the amino acid sequence as set forth in SEQ ID NO: 47, and the peptide chain II comprising the amino acid sequence as set forth in SEQ ID NO: 38.

In certain embodiments, the bispecific antibody comprises two peptide chains I and two peptide chains II.

In certain embodiments, the two peptide chains I comprised in the bispecific antibody are identical or different, and/or the two peptide chains II comprised in the bispecific antibody are identical or different.

In certain embodiments, the bispecific antibody comprises two identical peptide chains I and two identical peptide chains II.

The bispecific antibody of the present disclosure may be prepared by various methods known in the art, e.g., by recombinant genetic engineering techniques. For example, a DNA molecule encoding the peptide chain of the bispecific antibody of the present disclosure is obtained by chemical synthesis or PCR amplification. The resulting DNA molecule is inserted into an expression vector, which is then transfected into host cells. Subsequently, the transfected host cells are cultured under specific conditions to express the bispecific antibody of the present disclosure.

In another aspect, the present application provides an isolated nucleic acid molecule or set of nucleic acid molecules comprising a nucleotide sequence encoding the bispecific antibody as described above. According to codon degeneracy in the art, in certain embodiments, the nucleotide sequence may be substituted according to codon degeneracy. In certain embodiments, the nucleotide sequence is codon-optimized.

It is readily understood that the bispecific antibody of the present disclosure may consist of one or more polypeptide chains, and there is no limitation on the number of nucleic acid molecule chains in the isolated nucleic acid molecule or set of nucleic acid molecules encoding the bispecific antibody of the present disclosure.

In certain embodiments, the bispecific antibody of the present disclosure comprises the peptide chain I and the peptide chain II as described above, and the isolated nucleic acid molecule or set of nucleic acid molecules comprises a first nucleotide sequence encoding the peptide chain I of the bispecific antibody of the present disclosure and a second nucleotide sequence encoding the peptide chain II thereof, wherein the first nucleotide sequence and the second nucleotide sequence are present on the same or different nucleic acid molecules.

In another aspect, the present application provides a vector comprising the isolated nucleic acid molecule or set of nucleic acid molecules as described above.

In certain embodiments, the vector is a cloning vector or an expression vector.

In certain embodiments, the vector of the present disclosure is, e.g., a plasmid, a cosmid, a phage, a lentivirus, and the like. In certain embodiments, the vector is capable of expressing the antibody or antigen-binding fragment thereof of the present disclosure in a subject (e.g., a mammal, such as a human).

It is readily understood that the isolated nucleic acid molecule or set of nucleic acid molecules as described above may be present in the vector in any form. For example, when the isolated nucleic acid molecule or set of nucleic acid molecules comprises multiple nucleotide sequences encoding different peptide chains, the multiple nucleotide sequences may be located on the same vector or on different vectors. There is no limitation on the orientation, relative position, or connection mode of the multiple nucleotide coding sequences in the vector.

In certain embodiments, the bispecific antibody of the present disclosure comprises the peptide chain I and the peptide chain II as described above, and the vector comprises a first nucleotide sequence encoding the peptide chain I of the bispecific antibody of the present disclosure and a second nucleotide sequence encoding the peptide chain II thereof, wherein the first nucleotide sequence and the second nucleotide sequence are present on the same or different vector molecules. When the first nucleotide sequence and the second nucleotide sequence are present on different vector molecules, the vector of the present disclosure comprises a first vector comprising the first nucleotide sequence and a second vector comprising the second nucleotide sequence.

In another aspect, the present application provides a host cell comprising the isolated nucleic acid molecule or set of nucleic acid molecules, or the vector as described above.

The host cell may be a eukaryotic cell (e.g., a mammalian cell, insect cell, yeast cell) or a prokaryotic cell (e.g., *E. coli*). Suitable eukaryotic cells include, but are not limited to, NS0 cells, Vero cells, Hela cells, COS cells, CHO cells, ExpiCHO cells, HEK293 cells, Expi293 cells, BHK cells, and MDCKII cells. Suitable insect cells include, but are not limited to, Sf9 cells. In certain embodiments, the host cell of the present disclosure is a mammalian cell, e.g., CHO (e.g., CHO-EBNA, CHO-K1, CHO-S, CHO DXB11, ExpiCHO, CHO DG44).

In another aspect, the present application provides a method for preparing the bispecific antibody as described above, comprising culturing the host cell as described above under conditions allowing the expression of the bispecific antibody, and recovering the bispecific antibody from the cultured host cell culture.

The bispecific antibody of the present disclosure may be derivatized, e.g., linked to another molecule (e.g., another polypeptide or protein). Generally, derivatization (e.g., labeling) of the bispecific antibody will not adversely affect its binding to PD-1 and CD40 (particularly human PD-1 and human CD40). Therefore, the bispecific antibody of the present disclosure is also intended to include such derivatized forms. For example, the bispecific antibody of the present disclosure may be functionally linked (by chemical coupling, genetic fusion, non-covalent association, or other means) to one or more other molecular groups, e.g., a detection agent, a pharmaceutical agent, and/or a protein or polypeptide capable of mediating the binding of the bispecific antibody to another molecule (e.g., avidin or a polyhistidine tag).

As one of the derivatives of the antibody, the present disclosure provides a conjugate comprising the antibody or antigen-binding fragment thereof of the present disclosure and a conjugation moiety.

In certain embodiments, the conjugation moiety is selected from a therapeutic agent.

In certain embodiments, the conjugation moiety is selected from substances capable of improving the biological properties (e.g., increasing serum half-life) of the antibody.

In another aspect, the present application provides a pharmaceutical composition comprising the bispecific antibody as described above, or the isolated nucleic acid molecule or set of nucleic acid molecules, or the vector, or the host cell, and a pharmaceutically acceptable carrier and/or excipient.

In certain embodiments, the pharmaceutical composition further comprises an additional pharmaceutically active agent.

In certain embodiments, the additional pharmaceutically active agent is a drug having anti-tumor activity.
In certain embodiments, the bispecific antibody and the additional pharmaceutically active agent are provided as separate components or as a mixed component.

In another aspect, the present application provides use of the bispecific antibody as described above, or the isolated nucleic acid molecule or set of nucleic acid molecules, or the vector, or the host cell, or the pharmaceutical composition in the manufacture of a medicament for use in:
(1) enhancing immune cell activity *in vitro* or in a subject (e.g., a human or a monkey);
(2) enhancing an immune response in a subject (e.g., a human or a monkey);
(3) preventing and/or treating a tumor in a subject (e.g., a human or a monkey); and/or
(4) preventing and/or treating an infection in a subject (e.g., a human or a monkey).

In certain embodiments, the immune cell is a T cell, a B cell, a DC cell, a macrophage, and/or an NK cell.

In certain embodiments, the tumor is a solid tumor, such as melanoma (e.g., metastatic malignant melanoma), breast cancer, renal cancer (e.g., clear cell carcinoma), prostate cancer, bladder cancer, pancreatic cancer, lung cancer (e.g., non-small cell lung cancer), colon cancer, esophageal cancer, head and neck squamous cell carcinoma, liver cancer, ovarian cancer, cervical cancer, thyroid cancer, glioblastoma, or glioma.

In certain embodiments, the tumor is a hematologic malignancy, such as lymphoma or leukemia. In certain embodiments, the lymphoma is Hodgkin's lymphoma or non-Hodgkin's lymphoma; preferably, the non-Hodgkin's lymphoma is one or more of peripheral T-cell lymphoma, angioimmunoblastic T-cell lymphoma, EBV-positive NK/T-cell lymphoma (nasal type), and B-cell non-Hodgkin's lymphoma.

In certain embodiments, the infection is selected from a viral infection, a bacterial infection, a fungal infection, and a parasitic infection.

In certain embodiments, the subject is a mammal, e.g., a human or a monkey.

In certain embodiments, the bispecific antibody, the isolated nucleic acid molecule or set of nucleic acid molecules, the vector, the host cell, the conjugate, or the pharmaceutical composition is administered in combination with an additional pharmaceutically active agent, e.g., simultaneously, separately, or sequentially.

In certain embodiments, the additional pharmaceutically active agent is a drug having anti-tumor activity.

In another aspect, the present application provides a method for enhancing an immune response, and/or preventing and/or treating a tumor or infection in a subject; the method comprising: administering an effective amount of the bispecific antibody as described above, or the isolated nucleic acid molecule or set of nucleic acid molecules, or the vector, or the host cell, or the pharmaceutical composition to a subject in need thereof.

In certain embodiments, the tumor is a solid tumor, such as melanoma (e.g., metastatic malignant melanoma), breast cancer, renal cancer (e.g., clear cell carcinoma), prostate cancer, bladder cancer, pancreatic cancer, lung cancer (e.g., non-small cell lung cancer), colon cancer, esophageal cancer, head and neck squamous cell carcinoma, liver cancer, ovarian cancer, cervical cancer, thyroid cancer, glioblastoma, or glioma.

In certain embodiments, the tumor is a hematologic malignancy, such as lymphoma or leukemia. In certain embodiments, the lymphoma is Hodgkin's lymphoma or non-Hodgkin's lymphoma; preferably, the non-Hodgkin's lymphoma is one or more of peripheral T-cell lymphoma, angioimmunoblastic T-cell lymphoma, EBV-positive NK/T-cell lymphoma (nasal type), and B-cell non-Hodgkin's lymphoma.

In certain embodiments, the infection is selected from a viral infection, bacterial infection, fungal infection, and parasitic infection.

In certain embodiments, the subject is a mammal, e.g., a human or a monkey.

In certain embodiments, the bispecific antibody, isolated nucleic acid molecule or set of nucleic acid molecules, vector, host cell, conjugate, or pharmaceutical composition is administered in combination with an additional pharmaceutically active agent, e.g., simultaneously, separately, or sequentially.

In certain embodiments, the additional pharmaceutically active agent is a drug having anti-tumor activity.

In certain embodiments, the method further comprises administering to the subject a second therapy selected from surgery, chemotherapy, radiotherapy, immunotherapy, gene therapy, DNA therapy, RNA therapy, nanotherapy, viral therapy, adjuvant therapy, and any combination thereof; optionally, the second therapy may be applied simultaneously, separately, or sequentially with the bispecific antibody, isolated nucleic acid molecule or set of nucleic acid molecules, vector, host cell, conjugate, or pharmaceutical composition of the present application.

The bispecific antibody or the pharmaceutical composition of the present disclosure may be formulated into any dosage form known in the medical field, e.g., a tablet, a pill, a suspension, an emulsion, a solution, a gel, a capsule, a powder, a granule, an elixir, a lozenge, a suppository, an injection (including an injection solution, a sterile powder for injection, and a concentrated solution for injection), an inhalant, a spray, etc. The preferred dosage form depends on the intended mode of administration and therapeutic use. The bispecific antibody or pharmaceutical composition of the present disclosure should be sterile and stable under the conditions of manufacture and storage. A preferred dosage form is an injection. Such an injection may be a sterile injectable solution. For example, the sterile injectable solution may be prepared by incorporating a required dose of the bispecific antibody of the present disclosure in an appropriate solvent, and optionally incorporating other desired ingredients (including but not limited to, a pH adjusting agent, a surfactant, an adjuvant, an ionic strength enhancing agent, an isotonic agent, a preservative, a diluent, or any combination thereof), followed by filter sterilization. Furthermore, the sterile injectable solution may be prepared as a sterile lyophilized powder (e.g., by vacuum drying or freeze-drying) for ease of storage and use. Such a sterile lyophilized powder may be dispersed in a suitable carrier, such as sterile pyrogen-free water, before use.

Additionally, the bispecific antibody of the present disclosure may be presented in the pharmaceutical composition in a unit dosage form for ease of administration.

The bispecific antibody or pharmaceutical composition of the present disclosure may be administered by any suitable method known in the art, including but not limited to oral, buccal, sublingual, ocular, topical, parenteral, rectal, intrathecal, intracisternal, inguinal, intravesical, local (e.g., powder, ointment, or drops), or nasal routes. However, for many therapeutic uses, the preferred route/mode of administration is parenteral administration (e.g., intravenous injection, subcutaneous injection, intraperitoneal injection, intramuscular injection). The skilled artisan will appreciate that the route and/or mode of administration will vary depending upon the intended purpose. In a preferred embodiment, the bispecific antibody or pharmaceutical composition of the present disclosure is administered by intravenous infusion or injection.

The pharmaceutical composition of the present disclosure may include a "therapeutically effective amount" of the bispecific antibody of the present disclosure. A "therapeutically effective amount" refers to an amount sufficient to cure or at least partially arrest the disease and its complications in a patient already suffering from the disease. The therapeutically effective amount of the bispecific antibody of the present disclosure may vary depending upon factors such as the severity of the disease to be treated, the overall state of the patient's own immune system, the patient's general condition, such as age, weight, and sex, the mode of administration of the drug, and other concurrent treatments, etc.

In the present disclosure, the dosage regimen may be adjusted to provide the optimal desired response (e.g., therapeutic response). For example, either a single dose or several divided doses over a period of time may be administered, or the dose may be reduced or increased proportionally as indicated by the exigencies of the therapeutic situation.
In the present disclosure, the subject may be a mammal, e.g., a human.
In another aspect, the present application further provides a conjugate comprising the bispecific antibody as described above and a detectable label linked to the antibody or antigen-binding fragment thereof.

In certain embodiments, the detectable label is selected from an enzyme (e.g., horseradish peroxidase or alkaline phosphatase), a chemiluminescent reagent (e.g., acridinium esters, luminol and derivatives thereof, or ruthenium derivatives), a fluorescent dye (e.g., fluorescein or fluorescent protein), a radionuclide, or biotin.

In another aspect, the present application further provides a kit comprising the bispecific antibody or the conjugate as described above.

In certain embodiments, the kit comprises the conjugate as described above.

In certain embodiments, the kit comprises the bispecific antibody as described above, and a second antibody that specifically recognizes the antibody or antigen-binding fragment thereof. In certain embodiments, the second antibody further comprises a detectable label, e.g., an enzyme (e.g., horseradish peroxidase or alkaline phosphatase), a chemiluminescent reagent (e.g., acridinium esters, luminol and derivatives thereof, or ruthenium derivatives), a fluorescent dye (e.g., fluorescein or fluorescent protein), a radionuclide, or biotin.

In another aspect, the present application further provides a method for detecting the presence or level of PD-1 and/or CD40 in a sample, comprising using the bispecific antibody or conjugate as described above. In certain embodiments, the method is for therapeutic purposes, diagnostic purposes, or non-therapeutic and non-diagnostic purposes.

In certain embodiments, the method is an immunological assay, e.g., immunoblotting, enzyme immunoassay (e.g., ELISA), chemiluminescence immunoassay, fluorescence immunoassay, or radioimmunoassay.

In certain embodiments, the method comprises using the conjugate as described above.

In certain embodiments, the method comprises using the bispecific antibody as described above, and the method further comprises using a second antibody carrying a detectable label (e.g., an enzyme (e.g., horseradish peroxidase or alkaline phosphatase), a chemiluminescent reagent (e.g., acridinium esters, luminol and derivatives thereof, or ruthenium derivatives), a fluorescent dye (e.g., fluorescein or fluorescent protein), a radionuclide, or biotin) to detect the antibody or antigen-binding fragment thereof.

In certain embodiments, the method comprises: (1) contacting the sample with the bispecific antibody or conjugate of the present disclosure; (2) detecting the formation of an antigen-antibody immune complex or detecting the amount of the immune complex. The formation of the immune complex indicates the presence of PD-1 and/or CD40 or cells expressing PD-1 or CD40.

In another aspect, the present application provides use of the bispecific antibody or conjugate as described above in the manufacture of a detection reagent for detecting the presence or level of PD-1 and/or CD40 in a sample.

In certain embodiments, the detection reagent detects the presence or level of PD-1 and/or CD40 in a sample by the method for detecting the presence or level of PD-1 and/or CD40 in a sample as described above.

In certain embodiments, the sample is a cell sample from a subject (e.g., a mammal, preferably a human or a monkey).

### DEFINITIONS

In the present disclosure, unless otherwise specified, scientific and technical terms used herein have the meanings commonly understood by a person skilled in the art. Moreover, the virological, biochemical, and immunological laboratory procedures used herein are routine procedures widely used in their respective fields. Meanwhile, for a better understanding of the present disclosure, definitions and explanations of relevant terms are provided below.

When the terms "for example," "such as," "including," "comprising," or variations thereof are used herein, these terms will not be considered restrictive terms, but rather will be interpreted as meaning "but not limited to" or "not limited to."

Unless otherwise indicated herein or clearly contradicted by context, the terms "a," "an," and "the," and similar designations are to be construed to cover both the singular and the plural in the context of describing the present disclosure (especially in the context of the following claims).

As used herein, the term "antibody" is used in the broadest sense and encompasses various antibody structures, including but not limited to monoclonal antibodies, polyclonal antibodies, multispecific antibodies (e.g., bispecific antibodies), and antibody fragments, so long as they exhibit the desired antigen-binding activity. For example, an immunoglobulin molecule is composed of two pairs of polypeptide chains, each having one light chain (LC) and one heavy chain (HC). Antibody light chains are classified as kappa (κ) and lambda (λ) light chains. The heavy chains are classified as µ, δ, γ, α, or ε, and the isotype of the antibody as IgM, IgD, IgG, IgA, and IgE, respectively. Within the light and heavy chains, the variable and constant regions are joined by a "J" region of about 12 or more amino acids, and the heavy chains further comprise a "D" region of about 3 or more amino acids. Each heavy chain is composed of a heavy chain variable region (VH) and a heavy chain constant region (CH). The heavy chain constant region is composed of three domains, CH1, CH2, and CH3. Each light chain is composed of a light chain variable region (VL) and a light chain constant region (CL). The light chain constant region is composed of one domain, CL. The constant domain is not directly involved in the binding of an antibody to an antigen but exhibits various effector functions, such as mediating the binding of immunoglobulin to host tissues or factors, including various cells of the immune system (e.g., effector cells) and the first component (C1q) of the classical complement system. The VH and VL regions are also subdivided into regions of hypervariability, termed complementarity-determining regions (CDRs), interspersed with more conserved regions termed framework regions (FRs). Each VH and VL is composed of three CDRs and four FRs arranged from amino-terminus to carboxyl-terminus in the following order: FR1, CDR1, FR2, CDR2, FR3, CDR3, FR4. The variable regions (VH and VL) of each heavy/light chain pair form the antigen-binding site, respectively. The assignment of amino acids in each region or domain may follow the definition of Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

Herein, unless the context clearly indicates otherwise, when referring to the term "antibody," it includes not only intact antibody but also antigen-binding fragments of the antibody.

As used herein, the term "complementarity determining region" or "CDR" refers to the amino acid residues in the antibody variable region responsible for antigen binding. The precise boundaries of these amino acid residues can be defined according to various numbering systems known in the art, e.g., according to the definitions in the AbM numbering system (Martin ACR, Cheetham JC, Rees AR (1989) Modelling antibody hypervariable loops: A combined algorithm. Proc Natl Acad Sci USA 86:9268-9272), the MacCallum numbering system (MacCallum et al., (1996) J Mol Biol 262:732-745, see also e.g., Martin A. "Protein Sequence and Structure Analysis of Antibody Variable Domains," in Antibody Engineering, Kontermann and Dübel, eds., Chapter 31, pp. 422-439, Springer-Verlag, Berlin (2001)), the AHo numbering system (Honegger and Plückthun, A., J. Mol. Biol. 309:657-670 (2001)), or the IMGT numbering system (Lefranc et al., Dev. Comparat. Immunol. 27:55-77, 2003). For a given antibody, those skilled in the art will readily identify the CDRs defined by each numbering system. Moreover, the correspondence between different numbering systems is well known to those skilled in the art (see, e.g., Lefranc et al., Dev. Comparat. Immunol. 27:55-77, 2003).

In the present disclosure, the CDRs comprised in the antibody or antigen-binding fragment thereof of the present disclosure may be determined according to various numbering systems known in the art. In certain embodiments, the CDRs comprised in the antibody or antigen-binding fragment thereof of the present disclosure are determined by the Kabat, Chothia, MacCallum, IMGT, AHo, or AbM numbering system.

As used herein, the term "framework region" or "FR" residues refers to those amino acid residues in the variable region of an antibody other than the CDR residues as defined above.

The term "antibody" is not limited to an antibody produced by any specific method. For example, the antibody includes a recombinant antibody, a monoclonal antibody, and a polyclonal antibody. The antibody may be of different isotypes, e.g., IgG (e.g., IgG1, IgG2, IgG3, or IgG4 subtypes), IgA1, IgA2, IgD, IgE, or IgM antibody.

As used herein, the term "bispecific antibody" refers to an antibody having binding specificities for two different antigens (or epitopes), comprising two antibodies with binding specificities for different antigens (or epitopes). The antigen-binding domains are capable of binding to two different binding sites and/or target molecules. Each antigen-binding domain comprised in the bispecific antibody can be independently selected from a full-length antibody (e.g., an IgG antibody) or an antigen-binding fragment thereof (e.g., Fv, Fab, scFab, or scFv). In some cases, the respective antigen-binding domains are connected by a peptide linker.

As used herein, the term "Fc domain" means an antibody fragment formed by the disulfide bonding of the second and third constant regions of the first heavy chain of an antibody with the second and third constant regions of the second heavy chain. The Fc fragment of an antibody has various functions but does not participate in antigen binding.

As used herein, the term "scFv" refers to a single polypeptide chain comprising VL and VH domains, wherein the VL and VH are connected by a linker. Such scFv molecules can have the general structure: NH2-VL-linker-VH-COOH or NH2-VH-linker-VL-COOH. Suitable prior art peptide linkers consist of repeated GGGGS amino acid sequences or variants thereof. For example, a linker having the amino acid sequence (GGGGS)4 (SEQ ID NO: 37) can be used, but variants thereof can also be used. In some cases, a disulfide bond may also exist between the VH and VL of the scFv.

As used herein, the term "Fab fragment" means an antibody fragment consisting of VL, VH, CL, and CH1 domains, which typically consists of one peptide chain comprising VL and CL and another peptide chain comprising VH and CH1. However, those skilled in the art will understand that a Fab domain can be arranged according to the natural orientation described above, but may also comprise domain substitutions or exchanges that facilitate proper pairing of VH and VL (e.g., domain exchanges in the Crossmab format).

As used herein, the terms "monoclonal antibody," "monoclonal antibody," and "mAb" have the same meaning and are used interchangeably, and refer to an antibody or a fragment of an antibody derived from a population of highly homogeneous antibody molecules, i.e., a population of completely identical antibody molecules except for possible naturally occurring mutations. The monoclonal antibody has high specificity for a single epitope on an antigen. The polyclonal antibody, in contrast to the monoclonal antibody, typically comprises at least two or more different antibodies, which typically recognize different epitopes on the antigen. Furthermore, the modifier "monoclonal" only indicates that the antibody is characterized as being obtained from a highly homogeneous population of antibodies, and should not be construed as requiring preparation of the antibody by any particular method.

As used herein, the term "bispecific antibody" or "BsAb" refers to an antibody having two distinct binding domains that enable the bispecific antibody to simultaneously bind two different antigens or two different epitopes of the same antigen.

As used herein, the term "specifically binds/specific binding" refers to a non-random binding reaction between two molecules, such as the reaction between an antibody and the antigen to which it is directed. The strength or affinity of a specific binding interaction can be determined by the equilibrium dissociation constant (KD) or half maximal effective concentration (EC50) of the interaction.

The property of specific binding between two molecules can be determined using methods well known in the art. One method involves measuring the rates of formation and dissociation of the antigen-binding site/antigen complex. Both the "association rate constant" (kₐ or kₒₙ) and the "dissociation rate constant" (k_{dis} or k_{off}) can be calculated from the concentration and the actual rates of association and dissociation (see Malmqvist M, Nature, 1993, 361:186-187). The ratio of k_{dis}/kₒₙ is equal to the dissociation constant KD (see Davies et al., Annual Rev Biochem, 1990; 59:439-473). KD, kₒₙ, and k_{dis} values can be measured by any effective method. In certain embodiments, the dissociation constant may be measured using bioluminescence interferometry (e.g., the ForteBio Octet method). Alternatively, surface plasmon resonance technology (e.g., Biacore) or Kinexa may also be used to measure the dissociation constant.

As used herein, the term "vector" refers to a nucleic acid vehicle into which a polynucleotide may be inserted. When a vector enables expression of a protein encoded by the inserted polynucleotide, the vector is referred to as an expression vector. The vector may be introduced into a host cell by transformation, transduction, or transfection, allowing the genetic material elements carried to be expressed in the host cell. The vector is well known to those skilled in the art and includes, but is not limited to: a plasmid; a phagemid; a cosmid; a artificial chromosome; such as a yeast artificial chromosome (YAC), a bacterial artificial chromosome (BAC), or a P1-derived artificial chromosome (PAC); a phage such as a lambda phage or a M13 phage, and an animal virus, etc. The animal virus used as a vector includes, but is not limited to, a retrovirus (including lentivirus), an adenovirus, an adeno-associated virus, a herpesvirus (such as herpes simplex virus), a poxvirus, a baculovirus, a papillomavirus, and a papovavirus (e.g., SV40). A vector may contain a variety of expression-controlling elements, including but not limited to a promoter sequence, a transcription initiation sequence, an enhancer sequence, a selection element, and a reporter gene. Additionally, the vector may contain an origin of replication.

Expression and cloning vectors contain nucleic acid sequences that enable the vector to replicate in one or more selected host cells. Typically, in cloning vectors, this sequence is one that enables the vector to replicate independently of the host chromosomal DNA, and it includes an origin of replication or an autonomously replicating sequence. The term "expression vector" as used herein refers to a vector comprising a recombinant polynucleotide, which comprises an expression control sequence operably linked to a nucleotide sequence to be expressed. The expression vector comprises sufficient cis-acting elements for expression; other elements for expression may be provided by the host cell or an in vitro expression system. Expression vectors include all those known in the art, such as a cosmid, a plasmid (e.g., naked or contained in liposomes), and a virus (e.g., a lentivirus, a retrovirus, an adenovirus, and an adeno-associated virus).

As used herein, the term "host cell" refers to a cell into which a vector may be introduced, including, but not limited to, prokaryotic cells such as *Escherichia coli* or Bacillus *subtilis*, fungal cells such as yeast cells or *Aspergillus*, insect cells such as S2 *Drosophila* cells or Sf9, or animal cells such as fibroblasts, NS0 cells, Vero cells, Hela cells, COS cells, CHO cells (e.g., CHO-K1, CHO-S, CHO DXB11, ExpiCHO, CHO DG44 cells), ExpiCHO cells, HEK293 cells, Expi293 cells, BHK cells, and MDCKII cells.

As used herein, the term "identity" is used to refer to the sequence matching between two polypeptides or two nucleic acids. When a position in both of the two compared sequences is occupied by the same base or amino acid monomer subunit (e.g., a position in each of two DNA molecules is occupied by adenine, or a position in each of two polypeptides is occupied by lysine), the respective molecules are identical at that position. The "percent identity" between two sequences is a function of the number of matching positions shared by the two sequences divided by the number of positions compared × 100. For example, if 6 out of 10 positions in two sequences match, then the two sequences have 60% identity. For example, the DNA sequences CTGACT and CAGGTT share 50% identity (3 out of 6 total positions match). Typically, a comparison is made when aligning two sequences to produce maximum identity. Such alignment may be conveniently performed using, e.g., the method of Needleman et al. (1970) J. Mol. Biol. 48:443-453, which can be performed by computer programs such as the Align program (DNAstar, Inc.). The percent identity between two amino acid sequences can also be determined using the algorithm of E. Meyers and W. Miller (Comput. Appl Biosci., 4:11-17 (1988)), which has been incorporated into the ALIGN program (version 2.0), using a PAM120 weight residue table, a gap length penalty of 12, and a gap penalty of 4. Additionally, the percent identity between two amino acid sequences may be determined using the algorithm of Needleman and Wunsch (J Mol Biol. 48:444-453 (1970)) in the GAP program, which has been incorporated into the GCG software package (available at www.gcg.com), using the Blossum 62 matrix or PAM250 matrix, and a gap weight of 16, 14, 12, 10, 8, 6, or 4, and a length weight of 1, 2, 3, 4, 5, or 6.

The abbreviations for the twenty conventional amino acids mentioned herein follow conventional usage. See, e.g., Immunology-A Synthesis (2nd Edition, E. S. Golub and D. R. Gren, Eds., Sinauer Associates, Sunderland, Mass. (1991)), which is incorporated herein by reference. In the present disclosure, the terms "polypeptide" and "protein" have the same meaning and are used interchangeably. Furthermore, in the present disclosure, amino acids are generally represented by single-letter and three-letter abbreviations well known in the art. For example, alanine may be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), including but not limited to: a pH adjusting agent, a surfactant, an adjuvant, a ionic strength enhancer, a diluent, an agent for maintaining osmotic pressure, an agent for delaying absorption, a preservative. For example, the pH adjuster includes, but is not limited to, phosphate buffers. The surfactant includes, but is not limited to, cationic, anionic, or nonionic surfactants, such as Tween-80. The ionic strength enhancer includes, but is not limited to, sodium chloride. The preservative includes, but is not limited to, various antibacterial and antifungal agents, such as parabens, chlorobutanol, phenol, sorbic acid, etc. The agent for maintaining osmotic pressure includes, but is not limited to, sugars, NaCl, and the like. The agent for delaying absorption includes, but is not limited to, monostearates and gelatin. The diluent includes, but is not limited to, water, aqueous buffers (e.g., buffered saline), alcohols, and polyols (e.g., glycerol), etc. The stabilizer has the meaning commonly understood by those skilled in the art that are capable of stabilizing the desired activity of an active ingredient in a drug, including but not limited to sodium glutamate, gelatin, SPGA, sugars (e.g., sorbitol, mannitol, starch, sucrose, lactose, dextran, or glucose), amino acids (e.g., glutamic acid, glycine), proteins (e.g., dried whey, albumin, or casein), or degradation products thereof (e.g., lactalbumin hydrolysate), etc.

As used herein, the term "prophylaxis" refers to a method performed to prevent or delay the onset of a disease or disorder or symptom (e.g., tumor, pathogenic infection) in a subject. As used herein, the term "treatment" refers to a method performed to obtain a beneficial or desired clinical outcome. For the purposes of the present disclosure, beneficial or desired clinical outcomes include but are not limited to alleviating symptoms, reducing the extent of the disease, stabilizing (i.e., not worsening) the state of the disease, delaying or slowing the progression of the disease, ameliorating or alleviating the state of the disease, and relieving symptoms (whether partially or totally), whether detectable or undetectable. Furthermore, "treatment" may also refer to prolonged survival compared to the expected survival if not treated.

As used herein, the term "subject" refers to a mammal, e.g., a primate mammal, such as a human. In certain embodiments, the subject (e.g., human) has a tumor or a pathogenic infection, or is at risk of having such diseases.

As used herein, the term "effective amount" refers to an amount sufficient to obtain, or at least partially obtain, a desired effect. For example, an effective amount to prevent a disease (e.g., tumor, pathogenic infection) refers to an amount sufficient to prevent, arrest, or delay the occurrence of the disease (e.g., tumor, pathogenic infection). An effective amount to treat a disease refers to an amount sufficient to cure or at least partially arrest an existing disease or complications. Determination of such an effective amount is well within the capability of those skilled in the art. For example, the amount effective for therapeutic use will depend on the severity of the disease to be treated, the overall state of the immune system of the patient, the general condition of the patient, such as age, weight, and sex, the mode of administration of a drug, and other treatments administered concurrently, etc.

As used herein, the term "effector function" refers to those biological activities attributable to the Fc region of an antibody (either native sequence Fc region or amino acid sequence variant Fc region) and which are associated with the antibody, varying with the antibody isotype. Examples of antibody effector functions include, but are not limited to: Fc receptor binding affinity, antibody-dependent cell-mediated cytotoxicity (ADCC), complement-dependent cytotoxicity (CDC), antibody-dependent cellular phagocytosis (ADCP), cell surface receptors (e.g., B cell receptor), B cell activation, cytokine secretion, and half-life/clearance rate of antibody and antigen-antibody complex, etc. Methods for altering the effector functions of an antibody are known in the art, e.g., by introducing mutations into the Fc region.

### Beneficial Effects of the Invention

The present application provides a bispecific antibody capable of specifically binding to PD-1 and CD40. The bispecific antibody provided herein is capable of: blocking the PD-1/PD-L1 signaling pathway, activating the CD40 downstream signaling pathway (e.g., the NF-κB pathway) in a manner dependent on PD-1 binding, activating primary B cells, activating T cells in a mixed lymphocyte reaction system, and/or inhibiting tumor growth in vivo.

Furthermore, the bispecific antibody provided herein also possesses cross-binding activity with human CD40 and monkey CD40, as well as cross-binding activity with human PD-1 and monkey PD-1.

In certain embodiments, the bispecific antibody provided herein is capable of blocking both the binding of PD-1 to PD-L1 and the binding of CD40 to CD40L. In certain embodiments, the bispecific antibody provided herein is capable of blocking the binding of PD-1 to PD-L1 but does not block the binding of CD40 to CD40L.

The embodiments of the present disclosure will be described in detail below with reference to the accompanying drawings and examples, but it will be understood by those skilled in the art that the following drawings and examples are intended to illustrate the present disclosure solely and are not intended to limit the scope of the present disclosure. Various objects and advantageous aspects of the present disclosure will become apparent to those skilled in the art from the following detailed description of the drawings and preferred embodiments.

### BRIEF DESCRIPTION OF THE DRAWINGS

Fig. 1A shows a schematic structural diagram of the anti-PD1-anti-CD40 (non-blocker) bispecific antibody of the present disclosure (corresponding to "BsAb-1" in Table 2).
Fig. 1B shows a schematic structural diagram of the anti-PD1-anti-CD40 (blocker) bispecific antibody of the present disclosure (corresponding to "BsAb-2" in Table 2).
Fig. 1C shows a schematic structural diagram of the anti-PD1(pembro)xCD40 bispecific antibody of the present disclosure (corresponding to "BsAb-3" in Table 2).
Fig. 2A shows the affinity of the anti-PD1-anti-CD40 bispecific antibody of the present disclosure for human/monkey PD-1 protein.
Fig. 2B shows the affinity of the anti-PD1-anti-CD40 bispecific antibody of the present disclosure for human/monkey CD40 protein.
Fig. 2C shows the affinity of the anti-PD1(pembro)xCD40 bispecific antibody of the present disclosure for human PD-1/CD40 protein.
Figs. 3A-3I show the binding activity of the anti-PD1-anti-CD40 bispecific antibody of the present disclosure to CHO-S cells overexpressing human/monkey CD40 or PD-1 (or control CHO-S cells). Wherein, "PD1xCD40" in Fig. 3I corresponds to "BsAb-1" in Table 2, and "PD1(Pembro)xCD40" corresponds to "BsAb-3" in Table 2.
Figs. 4A-4B show the blocking activity of the anti-PD1-anti-CD40 bispecific antibody of the present disclosure on the binding of CD40 to CD40L.
Figs. 5A-5B show the blocking activity of the anti-PD1-anti-CD40 bispecific antibody of the present disclosure on the PD-L1 and PD-1 signaling pathway.
Figs. 6A-6D show the activity of the anti-PD1-anti-CD40 bispecific antibody of the present disclosure in activating CD40 dependent on PD-1 expression.
Figs. 7A-7D show the activity of the anti-PD1-anti-CD40 bispecific antibody of the present disclosure in activating primary B cells.
Figs. 8A-8B show the experimental results of the anti-PD1-anti-CD40 bispecific antibody of the present disclosure activating T cells to release IL-2 in a mixed lymphocyte reaction system. Wherein, "PD1xCD40" in Fig. 8B corresponds to "BsAb-1" in Table 2, "PD1(Pembro)xCD40" corresponds to "BsAb-3" in Table 2, and "anti-CD40 mAb" corresponds to ADI-55164.
Figs. 9A-9C show the half-life of the anti-PD1-anti-CD40 bispecific antibody of the present disclosure.
Fig. 10A shows the in vivo efficacy test results of the anti-PD1-anti-CD40 bispecific antibody of the present disclosure in a subcutaneous mixed inoculation A375 and human PBMC B-NDG B2M KO Plus mouse tumor model.
Fig. 10B shows the in vivo efficacy test results of the anti-PD1-anti-CD40 bispecific antibody of the present disclosure in a subcutaneous mixed inoculation B16F10 and human PBMC huPD-1/CD40 double KI C57 mouse tumor model.

Note: In the drawings of the present application, "PD1xCD40(ADI-55164)" or "PD1xCD40(non-blocker)" corresponds to "BsAb-1" in Table 2; "PD1xCD40(ADI-55147)" or "PD1xCD40(blocker)" corresponds to "BsAb-2" in Table 2; "PD1(pembro)xCD40(ADI-55164)" corresponds to "BsAb-3" in Table 2; "PD-1 mAb" or "anti-PD1" corresponds to ADI-54872; "CD40 mAb(blocker)" corresponds to ADI-55147; "CD40 mAb(non-blocker)" corresponds to ADI-55164.

### Sequence Information

Information on some of the sequences involved in the present disclosure is provided in Table 1 below.

**Table 1: Description of Sequences**

| SEQ ID NO: | Description | Sequence (CDR is defined according to the IMGT numbering system) |
|---|---|---|
| 1 | PD-1xCD40 (ADI55164)-HC | QVQLVQSGAEVKKPGASVKVSCKASGYTFTEYYIYWVRQA PGQGLEWIGGINPSNGGTNFNEKFKPRVTMTVDTSTSTAYM ELSSLRSEDTAVYYCTVRDFRFDKGFKYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPEAAGAPSVFLF PPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFFL YSKLTVDKSRWQQGNVFSCSVMHEALHNHYTQKSLSLSPG GGGGAGGGGAEVQLVESGGGLVQPGRSLRLSCAASGFTFD DYAMHWVRQAPGKCLEWVSGISWSSDSIHYADSVKGRFTIS RDNAKNSLYLQMNSLRAEDTAVYYCAKDPTATTGARGYFD LWGRGTLVTVSSGGGGSGGGGSGGGGSGGGGSDIQMTQSP SSVSASVGDRVTITCRASKVISSWLAWYQQKPGKAPKLLIY AASYLESGVPSRFSGSGSGTDFTLTISSLQPEDFATYYCQQHS SFPPTFGCGTKVEIK |
| 2 | PD-1xCD40 (ADI55147)-NC | QVQLVQSGAEVKKPGASVKVSCKASGYTFTEYYIYWVRQA PGQGLEWIGGINPSNGGTNFNEKFKPRVTMTVDTSTSTAYM ELSSLRSEDTAVYYCTVRDFRFDKGFKYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPEAAGAPSVFLF PPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFFL YSKLTVDKSRWQQGNVFSCSVMHEALHNHYTQKSLSLSPG GGGGAGGGGAQVQLVESGGGVVQPGRSLRLSCAASGFTFG SYGMHWVRQAPGKCLEWVAVISYEGVKKYYADSVKGRFTI SRDNSKNTLYLQMNSLRAEDTAVYYCARDTSRGHDIWGQG TMVTVSSGGGGSGGGGSGGGGSGGGGSEIVLTQSPGTLSLS PGERATLSCRASQSVSYDYLAWYQQKPGQAPRLLIYGASAR ANGIPDRFSGSGSGTDFTLTISRLEPEDFAVYYCQQYADYPPF TFGCGTKVEIK |
| 3 | Anti-PD1 (ADI-54872) VH | QVQLVQSGAEVKKPGASVKVSCKASGYTFTEYYIYWVRQA PGQGLEWIGGINPSNGGTNFNEKFKPRVTMTVDTSTSTAYM ELSSLRSEDTAVYYCTVRDFRFDKGFKYWGQGTLVTVSS |
| 4 | Anti-PD1 (ADI-54872) VL | EIVLTQSPATLSLSPGERATLSCRASKSVSTSGLNYVHWYQR KPGQAPRLLIYLGSYLDSGVPARFSGSGSGTDFTLTISSLEPE DFAVYYCQQSWELPLTFGGGTKVEIK |
| 5 | ADI-55164 VH (anti CD40) | EVQLVESGGGLVQPGRSLRLSCAASGFTFDDYAMHWVRQA PGKGLEWVSGISWSSDSIHYADSVKGRFTISRDNAKNSLYL QMNSLRAEDTAVYYCAKDPTATTGARGYFDLWGRGTLVTV SS |
| 6 | ADI-55147 VH (anti CD40) | QVQLVESGGGVVQPGRSLRLSCAASGFTFGSYGMHWVRQ APGKGLEWVAVISYEGVKKYYADSVKGRFTISRDNSKNTLY LQMNSLRAEDTAVYYCARDTSRGHDIWGQGTMVTVSS |
| 7 | ADI-55164 HCDR1 (IMGT) | GFTFDDYA |
| 8 | ADI-55164 HCDR2 (IMGT) | ISWSSDSI |
| 9 | ADI-55164 HCDR3 (IMGT) | AKDPTATTGARGYFDL |
| 10 | ADI-55147 HCDR1 (IMGT) | GFTFGSYG |
| 11 | ADI-55147 HCDR2 (IMGT) | ISYEGVKK |
| 12 | ADI-55147 HCDR3 (IMGT) | ARDTSRGHDI |
| 13 | Anti-PD1 (ADI-54872) HCDR1 (IMGT) | GYTFTEYY |
| 14 | Anti-PD1 (ADI-54872) HCDR2 (IMGT) | INPSNGGT |
| 15 | Anti-PD1 (ADI-54872) HCDR3 (IMGT) | TVRDFRFDKGFKY |
| 16 | Anti-PD1 (ADI-54872) LCDR1 (IMGT) | KSVSTSGLNY |
| 17 | Anti-PD1 (ADI-54872) LCDR2 (IMGT) | LGS |
| 18 | Anti-PD1 (ADI-54872) LCDR3 (IMGT) | QQSWELPLT |
| 19 | Heavy chain constant region | ASTKGPSVFPLAPSSKSTSGGTAALGCLVKDYFPEPVTVSW NSGALTSGVHTFPAVLQSSGLYSLSSVVTVPSSSLGTQTYICN VNHKPSNTKVDKKVEPKSCDKTHTCPPCPAPEAAGAPSVFL FPPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFFL YSKLTVDKSRWQQGNVFSCSVMHEALHNHYTQKSLSLSPG |
| 20 | Light chain constant region | |
| 21 | CP-870893 VH | QVQLVQSGAEVKKPGASVKVSCKASGYTFTGYYMHWVRQ APGQGLEWMGWINPDSGGTNYAQKFQGRVTMTRDTSISTA YMELNRLRSDDTAVYYCARDQPLGYCTNGVCSYFDYWGQ GTLVTVSS |
| 22 | CP-870893 VL | DIQMTQSPSSVSASVGDRVTITCRASQGIYSWLAWYQQKPG KAPNLLIYTASTLQSGVPSRFSGSGSGTDFTLTISSLQPEDFAT YYCQQANIFPLTFGGGTKVEIK |
| 23 | ADI-55164 VL | DIQMTQSPSSVSASVGDRVTITCRASKVISSWLAWYQQKPG KAPKLLIYAASYLESGVPSRFSGSGSGTDFTLTISSLQPEDFAT YYCQQHSSFPPTFGGGTKVEIK |
| 24 | ADI-55147 VL | EIVLTQSPGTLSLSPGERATLSCRASQSVSYDYLAWYQQKPG QAPRLLIYGASARANGIPDRFSGSGSGTDFTLTISRLEPEDFA VYYCQQYADYPPFTFGGGTKVEIK |
| 25 | ADI-55164 LCDR1 (IMGT) | KVISSW |
| 26 | ADI-55164 LCDR2 (IMGT) | AAS |
| 27 | ADI-55164 LCDR3 (IMGT) | QQHSSFPPT |
| 28 | ADI-55147 LCDR1 (IMGT) | QSVSYDY |
| 29 | ADI-55147 LCDR2 (IMGT) | GAS |
| 30 | ADI-55147 LCDR3 (IMGT) | QQYADYPPFT |
| 31 | ADI-55164 VH (G44C substitution mutated) | EVQLVESGGGLVQPGRSLRLSCAASGFTFDDYAMHWVRQA PGKCLEWVSGISWSSDSIHYADSVKGRFTISRDNAKNSLYLQ MNSLRAEDTAVYYCAKDPTATTGARGYFDLWGRGTLVTVS S |
| 32 | ADI-55147 VH (G44C substitution mutated) | QVQLVESGGGVVQPGRSLRLSCAASGFTFGSYGMHWVRQ APGKCLEWVAVISYEGVKKYYADSVKGRFTISRDNSKNTLY LQMNSLRAEDTAVYYCARDTSRGHDIWGQGTMVTVSS |
| 33 | ADI-55164 VL (G100C substitution mutated) | DIQMTQSPSSVSASVGDRVTITCRASKVISSWLAWYQQKPG KAPKLLIYAASYLESGVPSRFSGSGSGTDFTLTISSLQPEDFAT YYCQQHSSFPPTFGCGTKVEIK |
| 34 | ADI-55147 VL (G102C substitution mutated) | EIVLTQSPGTLSLSPGERATLSCRASQSVSYDLAWYQQKPG QAPRLLIYGASARANGIPDRFSGSGSGTDFTLTISRLEPEDFA VYYCQQYADYPPFTFGCGTKVEIK |
| 35 | LC of bispecific antibody PD-1xCD40 | EIVLTQSPATLSLSPGERATLSCRASKSVSTSGLNYVHWYQR KPGQAPRLLIYLGSYLDSGVPARFSGSGSGTDFTLTISSLEPE DFAVYYCQQSWELPLTFGGGTKVEIKRTVAAPSVFIFPPSDE QLKSGTASVVCLLNNFYPREAKVQWKVDNALQSGNSQESV TEQDSKDSTYSLSSTLTLSKADYEKHKVYACEVTHQGLSSP VTKSFNRGEC |
| 36 | Linker1 | GGGGAGGGGA |
| 37 | Linker2 | GGGGSGGGGSGGGGSGGGGS |
| 38 | PD1 (pembro)xCD4 0 (ADI55164)-H C | EVQLVQSGVEVKKPGASVKVSCKASGYTFTNYYMYWVRQ APGQGLEWMGGINPSNGGTNFNEKFKNRVTLTTDSSTTTAY MELKSLQFDDTAVYYCARRDYRFDMGFDYWGQGTTVTVS SASTKGPSVFPLAPSSKSTSGGTAALGCLVKDYFPEPVTVSW NSGALTSGVHTFPAVLQSSGLYSLSSVVTVPSSSLGTQTYICN VNHKPSNTKVDKKVEPKSCDKTHTCPPCPAPEAAGAPSVFL FPPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFFL YSKLTVDKSRWQQGNVFSCSVMHEALHNHYTQKSLSLSPG GGGGAGGGGAEVQLVESGGGLVQPGRSLRLSCAASGFTFD DYAMHWVRQAPGKCLEWVSGISWSSDSIHYADSVKGRFTIS RDNAKNSLYLQMNSLRAEDTAVYYCAKDPTATTGARGYFD LWGRGTLVTVSSGGGGSGGGGSGGGGSGGGGSDIQMTQSP SSVSASVGDRVTITCRASKVISSWLAWYQQKPGKAPKLLIY AASYLESGVPSRFSGSGSGTDFTLTISSLQPEDFATYYCQQHS SFPPTFGCGTKVEIK |
| 39 | Anti- PD1 (pembro) VH | EVQLVQSGVEVKKPGASVKVSCKASGYTFTNYYMYWVRQ APGQGLEWMGGINPSNGGTNFNEKFKNRVTLTTDSSTTTAY MELKSLQFDDTAVYYCARRDYRFDMGFDYWGQGTTVTVS S |
| 40 | Anti- PD1 (pembro) VL | |
| 41 | Anti- PD1 (pembro) HCDR1 (IMGT) | GYTFTNYY |
| 42 | Anti- PD1 (pembro) HCDR2 (IMGT) | INPSNGGT |
| 43 | Anti- PD1 (pembro) HCDR3 (IMGT) | ARRDYRFDMGFDY |
| 44 | Anti- PD1 (pembro) LCDR1 (IMGT) | KGVSTSGYSY |
| 45 | Anti- PD1 (pembro) LCDR2 (IMGT) | LAS |
| 46 | Anti- PD1 (pembro) LCDR3 (IMGT) | QHSRDLPLT |
| 47 | PD1 (pembro)xCD4 0 (ADI55164)-L C | EIVLTQSPATLSLSPGERATLSCRASKGVSTSGYSYLHWYQQ KPGQAPRLLIYLASYLESGVPARFSGSGSGTDFTLTISSLEPE DFAVYYCQHSRDLPLTFGGGTKVEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPV TKSFNRGEC |
| 48 | YH008 HC | EVQLVESGGGLVQPGGSLRLSCAASGFTFSSYSMSWVRQAP GKRLEWVAHISSGGSSTYYPDTVKGRFTISRDNSKNTLYLQ MNSLRAEDTAMYYCTRQFYYGSSFWYFDVWGAGTTVTVS SASTKGPSVFPLAPSSKSTSGGTAALGCLVKDYFPEPVTVSW NSGALTSGVHTFPAVLQSSGLYSLSSVVTVPSSSLGTQTYICN VNHKPSNTKVDKKVEPKSCDKTHTCPPCPAPEAAGGPSVFL FPPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSREEMTKNQVS LTCLVKGFYPSDIAVEWESNGQPENNYKTTPPVLDSDGSFFL YSKLTVDKSRWQQGNVFSCSVMHEALHNHYTQKSLSLSPG GGGGSGGGGSGGGGSDVVMTQSPLSLPVTLGQPASISCRSS QSLLHSNGNTYLHWYQQRPGQSPNHLIYQVSNRFSGVPDRF SGSGSGTDFTLKISRVEAEDVGVYFCSQTTHVPWTFGCGTK VEIKGGGGSGGGGSGGGGSGGGGSQVQLVQSGAEVKKPG ASVKLSCKASGYTFISYYIYWVKQAPGQCLEWIGGINPRNG GTNFNEKFKSRATLTVDTSISTAYMELSRLRSEDTAVYYCTR HGNGVYWGQGTTLTVSS |
| 49 | YH008 LC | DNVLTQSPATLSVSPGERATLSCKASQSVDFDGDSYMNWYQ QKPGQPPRLLIYAASNLESGIPARFSGSGSGTDFTLTIS SVEPE DFATYYCQQSNEDPPTFGGGTKVEIKRTVAAPSVFIFPPSDEQ LKSGTASVVCLLNNFYPREAKVQWKVDNALQSGNSQESVT EQDSKDSTYSLSSTLTLSKADYEKHKVYACEVTHQGLSSPV TKSFNRGEC |

### DETAILED DESCRIPTION

The present disclosure will now described with reference to the following Examples intended to illustrate, but not to limit, the present disclosure.

Unless otherwise specified, the experiments and methods described in the examples were performed substantially according to conventional methods well known in the art and described in various references. For example, conventional techniques in immunology, biochemistry, chemistry, molecular biology, microbiology, cell biology, genomics, and recombinant DNA used in the present disclosure may be found in Sambrook, Fritsch, and Maniatis, MOLECULAR CLONING: A LABORATORY MANUAL, 2nd edition (1989); CURRENT PROTOCOLS IN MOLECULAR BIOLOGY (F.M. Ausubel et al., eds., (1987)); the METHODS IN ENZYMOLOGY series (Academic Press, Inc.); PCR 2: A PRACTICAL APPROACH (M.J. MacPherson, B.D. Hames, and G.R. Taylor, eds. (1995)), and ANIMAL CELL CULTURE (R.I. Freshney, ed. (1987)).

Furthermore, where conditions are not specified in the Examples, the procedures are carried out according to conventional conditions or conditions recommended by the manufacturer. The reagents or instruments used, if the manufacturer is not indicated, are conventional products commercially available. It will be appreciated by those skilled in the art that the Examples illustrate the present disclosure by way of example and are not intended to limit the scope of the claimed inventions. All publications and other references mentioned herein are incorporated by reference in their entirety.

### Example 1. Cloning and Expression of Bispecific Antibodies

### 1.1 Structure of Antibody Constructs

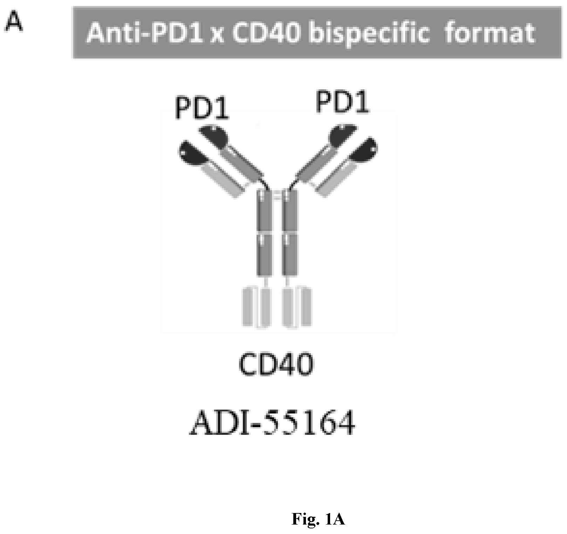

In this example, 2 anti-PD-1/CD40 bispecific antibodies were constructed, namely:
PD1xCD40(non-blocker, ADI-55164): It is composed of an anti-CD40 antibody having a non-blocker effect (i.e., anti-CD40 antibody ADI-55164 which has no blocking effect on the binding of CD40 and its ligand CD40L) and an anti-PD-1 antibody (ADI-54872), the structural schematic diagram of which is shown in Fig. 1A. The heavy chain of the bispecific antibody has the amino acid sequence as set forth in SEQ ID NO: 1, and the light chain has the amino acid sequence as set forth in SEQ ID NO: 35; wherein the bispecific antibody comprises the heavy chain and light chain of the anti-PD-1 antibody (ADI-54872), the Fc region comprised in the heavy chain of the anti-PD-1 antibody is an Fc region derived from human IgG1 (into which the L234AL235AL237A mutation was introduced to reduce Fc effector functions), and a single-chain variable fragment (scFv) of the anti-CD40 monoclonal antibody ADI-55164 is linked to the C-terminus of the heavy chain.

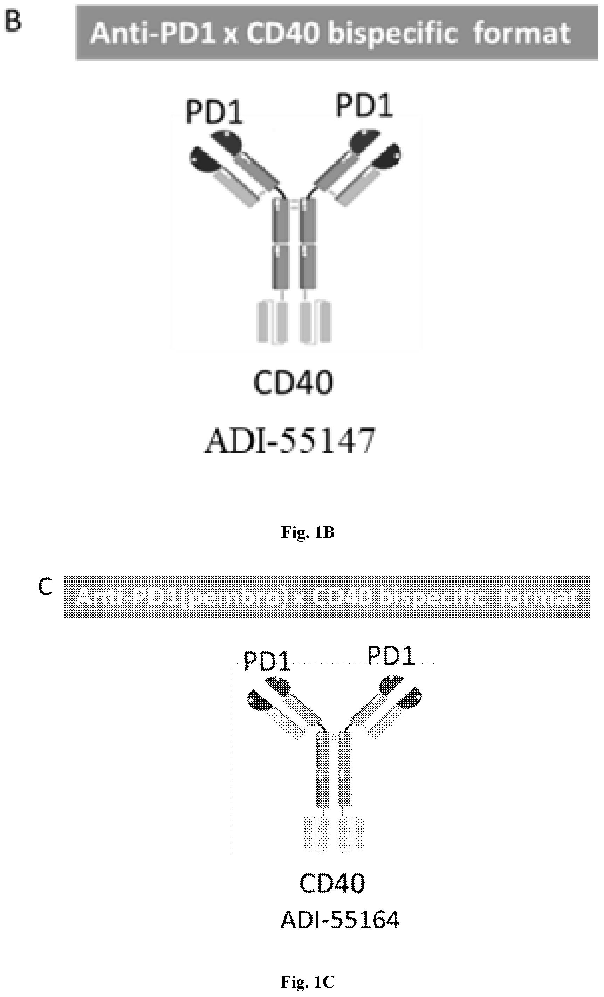

PD1xCD40(blocker, ADI-55147): It is composed of an anti-CD40 antibody having a blocker effect (i.e., anti-CD40 antibody ADI-55147 which has a blocking effect on the binding of CD40 and its ligand CD40L) and an anti-PD-1 antibody (ADI-54872), the structural schematic diagram of which is shown in Fig. 1B. The heavy chain of the bispecific antibody has the amino acid sequence as set forth in SEQ ID NO: 2, and the light chain has the amino acid sequence as set forth in SEQ ID NO: 35. The bispecific antibody comprises the heavy chain and light chain of the anti-PD-1 antibody (ADI-54872), the Fc region comprised in the heavy chain of the anti-PD-1 antibody is an Fc region derived from human IgG1 (into which the L234AL235AL237A mutation was introduced to reduce Fc effector functions), and a single-chain variable fragment (scFv) of the anti-CD40 monoclonal antibody ADI-55147 is linked to the C-terminus of the heavy chain.

PD1(pembro)xCD40(non-blocker, ADI-55164): It is composed of an anti-CD40 antibody having a non-blocker effect (i.e., an anti-CD40 antibody that has no blocking effect on the binding of CD40 and its ligand CD40L) and an anti-PD-1 antibody (Pembrolizumab, also referred to herein as pembro, with heavy chain variable region and light chain variable region amino acid sequences as set forth in SEQ ID NOs: 39 and 40, respectively), the structural schematic diagram of which is shown in Fig. 1C. The heavy chain of the bispecific antibody has the amino acid sequence as set forth in SEQ ID NO: 38, and the light chain has the amino acid sequence as set forth in SEQ ID NO: 47. The bispecific antibody comprises the heavy chain and light chain of the anti-PD-1 antibody (Pembrolizumab), the Fc region comprised in the heavy chain of the anti-PD-1 antibody is an Fc region derived from human IgG1 (into which the L234AL235AL237A mutation was introduced to reduce Fc effector functions), and a single-chain variable fragment (scFv) of the anti-CD40 monoclonal antibody ADI-55164 is linked to the C-terminus of the heavy chain.

The structure/sequence information of the above three bispecific antibodies is shown in Table 2.

**Table 2. Bispecific Antibody Structure/Sequence Information**

| Bispecific Antibody | Heavy chain | | | | | | Light chain | |
|---|---|---|---|---|---|---|---|---|
| | (From N- to C-terminus) | | | | | | (From N- to C-terminus) | |
| PD1xCD40 (non-blocker) (BsAb-1) | Anti-PD1 (ADI-54872) VH | CH | Linker1 | ADI55164 VH | Linker2 | ADI55164 VL | Anti-PD1 (ADI-54872) VL | CL |
| | SEQ ID NO: 3 | SEQ ID NO: 19 | SEQ ID NO: 36 | SEQ ID NO: 31 | SEQ ID NO: 37 | SEQ ID NO: 33 | SEQ ID NO: 4 | SEQ ID NO: 20 |
| PD1xCD40 (blocker) (BsAb-2) | Anti-PD1(ADI-54872) VH | CH | Linker1 | ADI55147 VH | Linker2 | ADI55147 VL | Anti-PD1(ADI-54872) VL | CL |
| | SEQ ID NO: 3 | SEQ ID NO: 19 | SEQ ID NO: 36 | SEQ ID NO: 32 | SEQ ID NO: 37 | SEQ ID NO: 34 | SEQ ID NO: 4 | SEQ ID NO: 20 |
| PD1(pembro)xCD40 (BsAb-3) | Anti- PD1(pembro) VH | CH | Linker1 | ADI55164 VH | Linker2 | ADI55164 VL | Anti-PD1(pembro) VL | CL |
| | SEQ ID NO: 39 | SEQ ID NO: 19 | SEQ ID NO: 36 | SEQ ID NO: 31 | SEQ ID NO: 37 | SEQ ID NO: 33 | SEQ ID NO : 40 | SEQ ID NO: 20 |

### 1.2 Gene Cloning and Protein Preparation

The above gene fragments were constructed into a pCDNA3.1 vector. Using the ExpiCHO^{™} Expression System kit (purchased from Thermo), the medium-scale prepared fusion protein expression plasmid was transfected into Expi-CHO cells according to the manufacturer's instructions. After 5 days of cell culture, the supernatant was collected, and the target protein was purified by affinity chromatography using Protein A magnetic beads (purchased from GenScript). The magnetic beads were resuspended in an appropriate volume (at 1-4 times the volume of the beads) of Binding buffer (PBS + 0.1% Tween 20, pH 7.4) and added to the sample to be purified. The mixture was incubated for 1 hour at room temperature with gentle shaking. The sample was placed on a magnetic rack (purchased from Beaver), the supernatant was discarded, and the magnetic beads were washed three times with Binding buffer. Elution buffer (0.1 M sodium citrate, pH 3.2) was added at 3-5 times the volume of the beads and shaken at room temperature for 5-10 min. The tube was placed back on the magnetic rack, and the Elution buffer was collected and transferred to a collection tube containing Neutralization buffer (1 M Tris, pH 8.54) and mixed to complete the preparation.

### Example 2: Affinity Detection of Bispecific Antibodies at the Protein Level

ForteBio affinity assay was performed according to established methods (Estep, P et al., High throughput solution Based measurement of antibody-antigen affinity and epitope binning. MAbs, 2013. 5(2): p. 270-8). Briefly, the sensor was equilibrated offline in assay buffer for 30 min, then detected online for 60 s to establish a baseline. The purified antibodies obtained as described above were loaded online onto the AHQ sensor. The sensor was then exposed to 100 nM antigen for 5 min, after which the sensor was transferred to PBS for dissociation for 5 min. Kinetic analysis was performed using a 1:1 binding model. YH008 (see Patent Application WO2022078357A1) served as a control anti-PD-1/CD40 bispecific antibody, with its heavy chain and light chain amino acid sequences as set forth in SEQ ID NOs: 48 and 49, respectively.

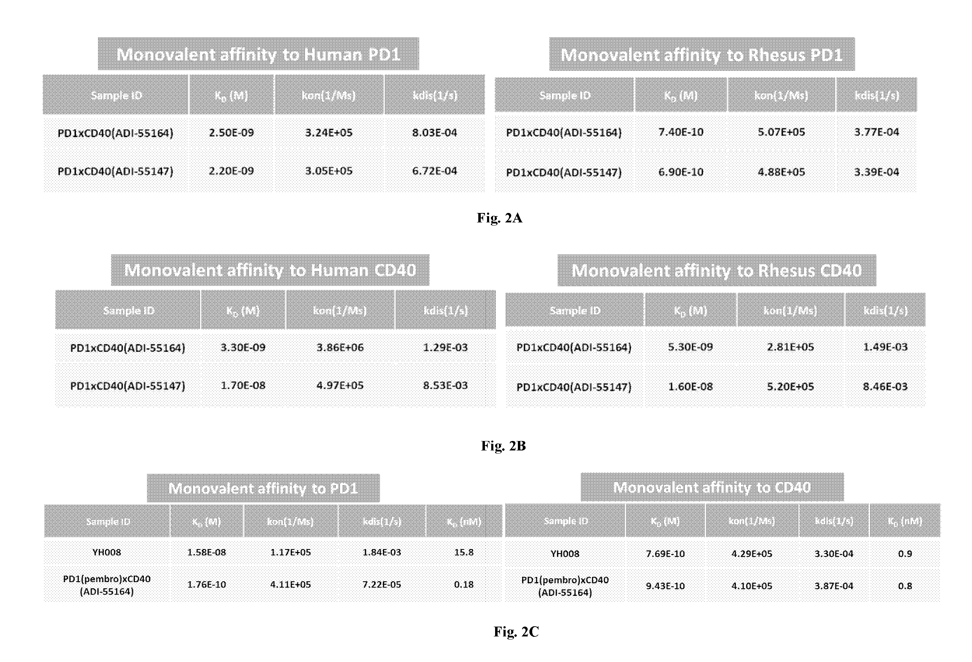

Results are shown in Figs. 2A-2C. The results show that all bispecific antibodies were capable of binding to human PD-1 and monkey PD-1, as well as human CD40 and monkey CD40, with strong affinity.

### Example 3: Binding of Bispecific Antibodies at the Cellular Level

CHO-S cells overexpressing human or cynomolgus monkey CD40 and PD-1 were generated by transfecting pCHO1.0 vectors (Invitrogen) with human CD40 and PD-1 cDNA, or cynomolgus monkey CD40 and PD-1 cDNA cloned into the MCS, followed by selection under pressure. Expanded-cultured CHO-hCD40 and CHO-hPD-1 cells, and CHO-cynoCD40 and CHO-cynoPD-1 cells were adjusted to a cell density of 2 × 10⁶ cells/ml, and was added to a 96-well flow cytometry plate at 100 µL/well, and centrifuged for future use. The purified bispecific antibodies were diluted with PBS, starting from 200 nM with 3-fold serial dilution for a total of 12 points. 100 µL/well of the diluted samples were added to the 96-well flow plate containing the cells, incubated at 4°C for 30 min, and washed twice with PBS. Goat Anti-Human IgG-Fc (PE) (purchased from Abcam, ab98596) diluted 100-fold in PBS was added at 100 µL/well, incubated at 4°C for 30 min, and washed twice with PBS. Cells were resuspended in PBS at 100 µL/well and analyzed on a CytoFlex (Beckman) flow cytometer, and the corresponding MFI was calculated. CP-870893 served as an anti-CD40 positive control antibody, with its heavy chain variable region and light chain variable region amino acid sequences as set forth in SEQ ID NOs: 21 and 22, respectively; CD40 mAb was ADI-55164.

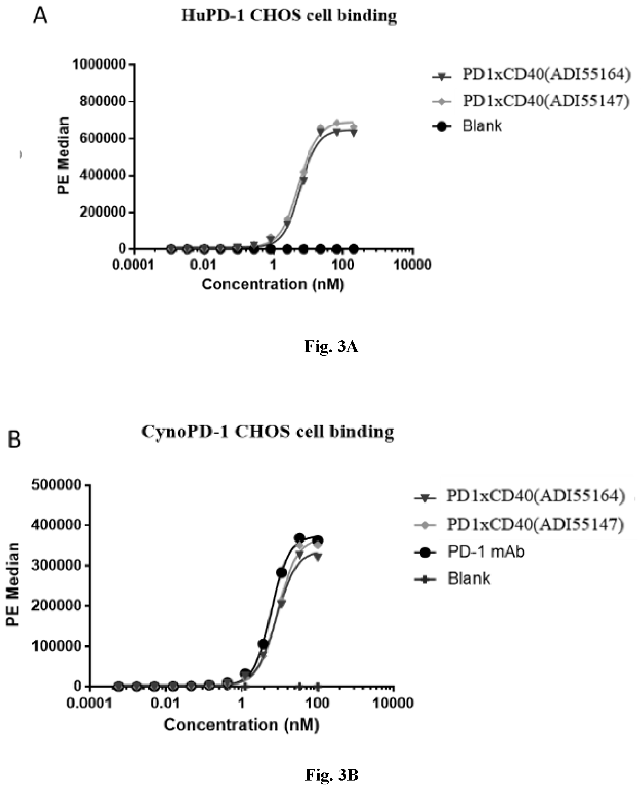

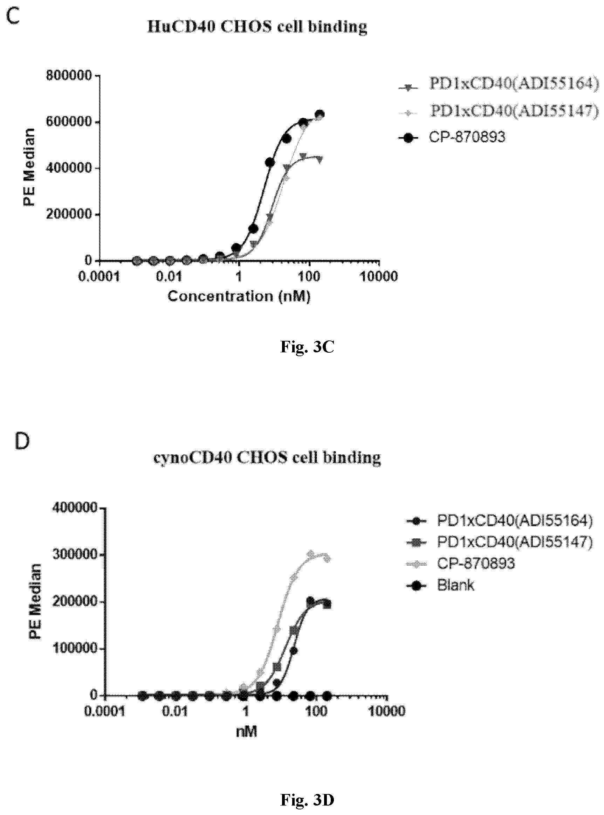

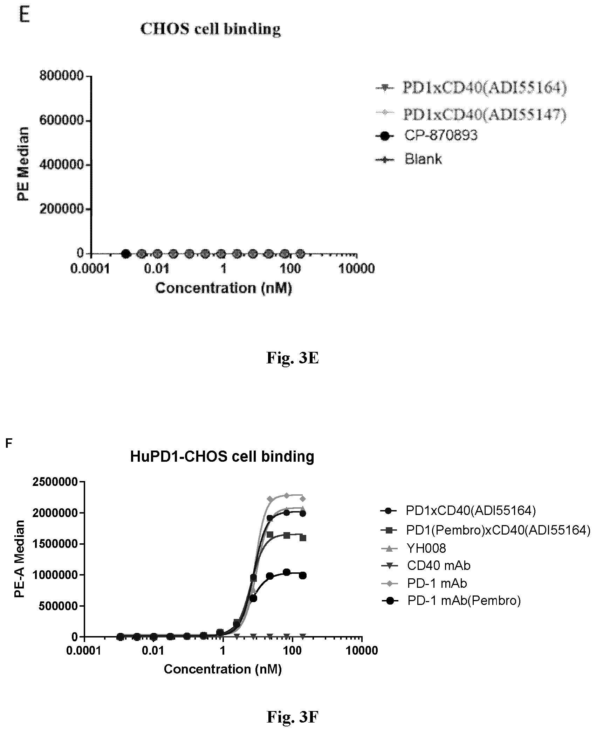

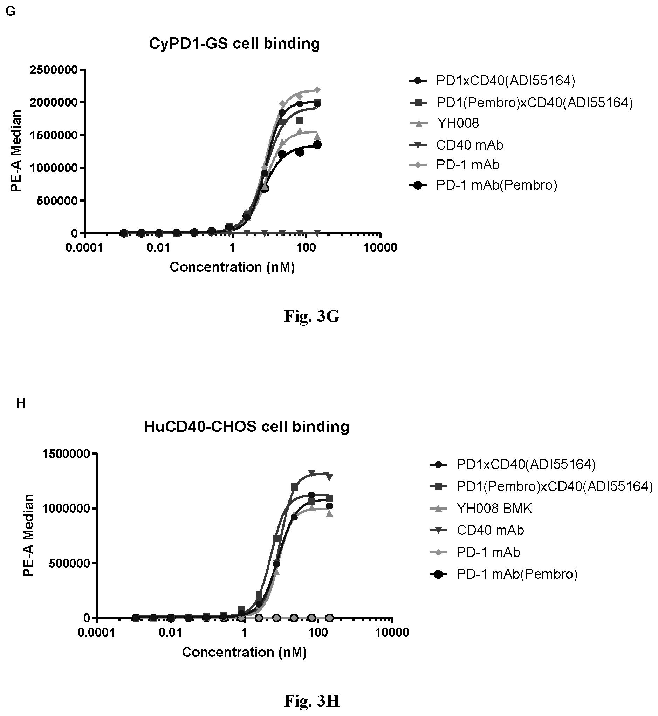

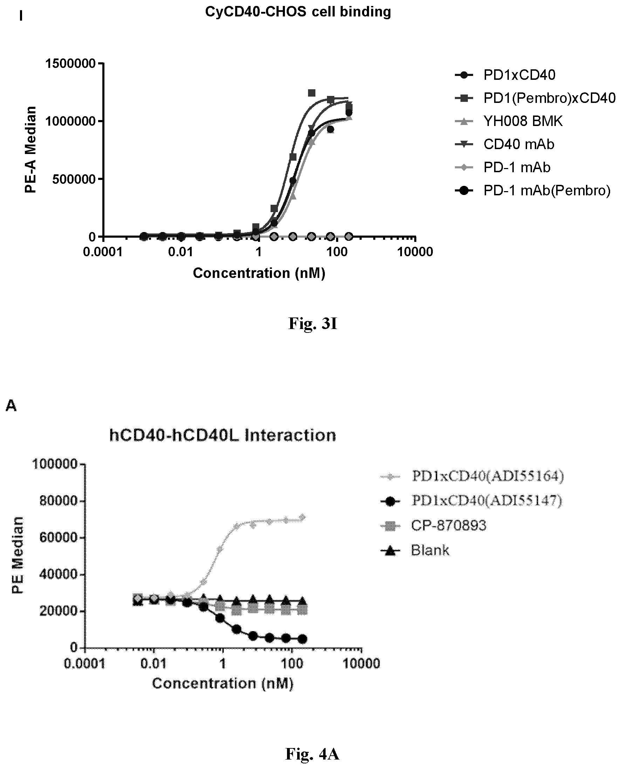

In the assay experiments according to the above method, the results are shown in Figs. 3A-3I. The results show that the purified samples of the bispecific antibodies shown in Table 2 have binding activity to CHO-S cells overexpressing human/monkey CD40 or PD-1, while having no significant non-specific binding to CHO-S cells that do not express PD-1 and CD40.

### Example 4: Blocking of CD40 Binding to CD40L by Bispecific Antibodies

The expanded Raji cells were adjusted to a cell density of 2 × 10⁶ cells/ml, and was added to a 96-well flow cytometry plate at 100 µL/well, and centrifuged for future use. The purified bispecific antibodies were diluted with PBS, starting from 200 nM with 3-fold serial dilution for a total of 12 points. 60 µL/well of the diluted samples were added to a 96-well sample dilution plate, together with 60 µL/well of biotin-labeled human CD40L protein (purchased from AcroBiosystems) to a final concentration of 500 ng/ml. The sample was co-incubated at 4°C for 30 minutes. The co-incubated sample was added at 100 µL/well to the 96-well flow plate containing the cells, incubated at 4°C for 30 minutes, and washed twice with PBS. SA-PE antibody (purchased from Biolegend) diluted 10,000-fold in PBS was added at 100 µL/well, incubated at 4°C for 30 minutes, and washed twice with PBS. Cells were resuspended in PBS at 100 µL/well and analyzed on a CytoFlex (Beckman) flow cytometer, and the corresponding MFI was calculated.

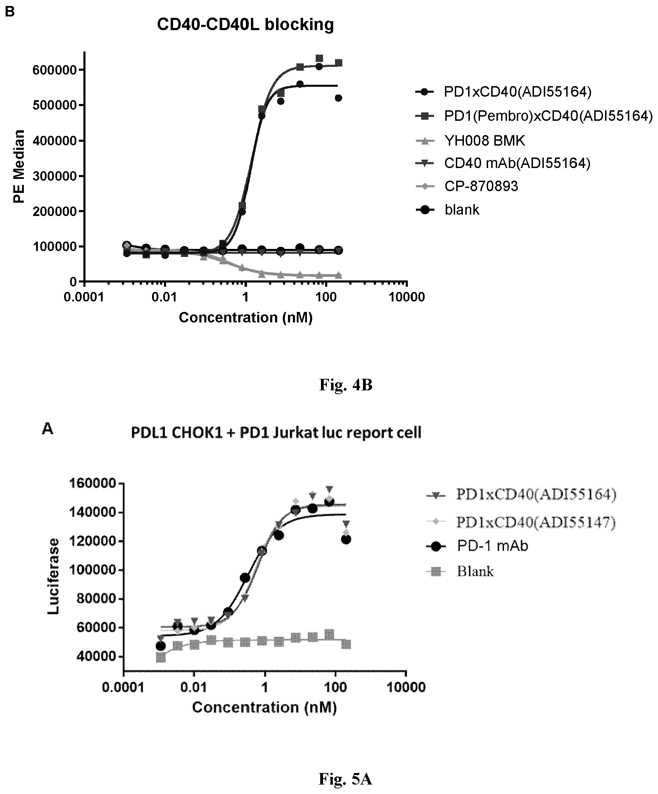

Results are shown in Figs. 4A-4B. The results show that one of the bispecific antibodies of different structures of the present disclosure, PD1xCD40(ADI-55147), can block the binding of CD40 to CD40L, while the other, PD1xCD40(ADI-55164), does not block the binding of CD40 to CD40L. PD1(pembro)xCD40(ADI-55164) also does not block CD40 binding to CD40L.

### Example 5: Blocking of PD-L1 and PD-1 Signaling Pathway by Bispecific Antibodies

CHO-K1-PD-L1 functional cells were harvested by digestion, and cell density was adjusted. 100 µL/well of the cells were added to a 96-well white opaque plate and cultured overnight to adhere. The next day, Jurkat-PD-1 effector cell suspension was prepared, and the test samples were serially diluted with reaction medium. The white opaque plate was removed, and the culture supernatant was aspirated. 40 µL/well of the diluted samples was added to the plate, along with 40 µL/well of Jurkat-PD-1-luc effector cell suspension. The plate was placed in a 37°C, 5% CO₂ incubator and cultured for 6 h. During this period, Bio-Glo^{™} reagent was allowed to equilibrate to room temperature. After the culture was complete, the cells were removed and allowed to equilibrate at room temperature for 5 min, and 80 µL/well of Bio-Glo^{™} reagent was added. Fluorescence signal values were read using a multi-mode microplate reader. The activity of PD1xCD40 bispecific antibody molecules in blocking the PD-1/PD-L1 signaling pathway was detected using CHO-K1 hPD-L1 + Jurkat hPD-1 NFAT-luciferase fluorescence reporter cells.

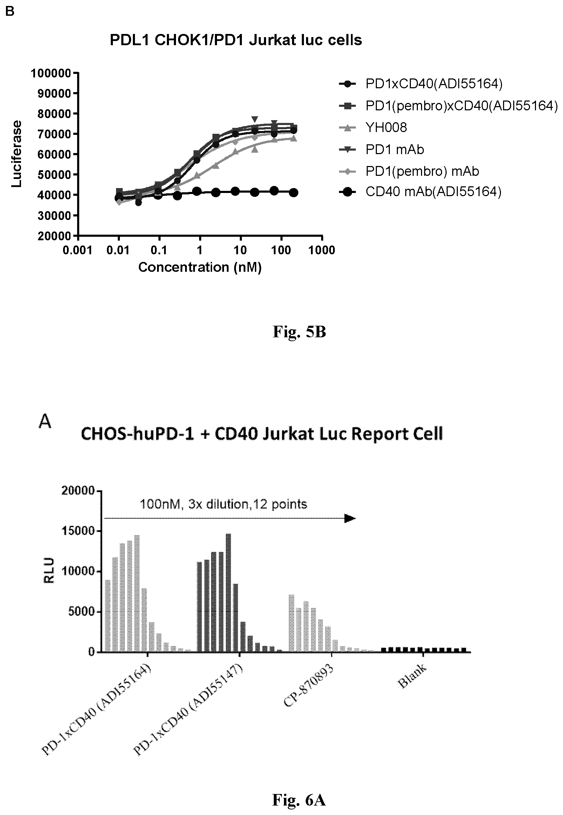

Results are shown in Figs. 5A-5B. The results show that the bispecific antibodies can block the PD-1/PD-L1 signaling pathway *in vitro*, with blocking activity comparable to that of the PD-1 monoclonal antibody.

### Example 6: Detection of CD40 Signaling Activation by Bispecific Antibodies Dependent on PD-1 Expression

The plasmids encoding human CD40 and plasmids encoding NF-κB luciferase reporter gene (purchased from Promega) were co-transfected into Jurkat cells to obtain huCD40 Jurkat-NF-κB cells. Expanded huCD40 Jurkat-NF-κB cells were resuspended in RPMI 1640 complete medium to 2 × 10⁶ cells/mL. The antibodies were diluted in medium, starting from 200 nM with 3-fold serial dilution, and 50 µL/well was added to sterile 96-well white opaque plates (purchased from Nunc). Target cells were added at 50 µL/well and incubated for 30 min. The huCD40-Jurkat-NFκB cells containing OKT-3 were added at 50 µL/well to a final concentration of 0.1 µg/mL and incubated overnight at 37°C, 5% CO₂. Luciferase signal was detected.

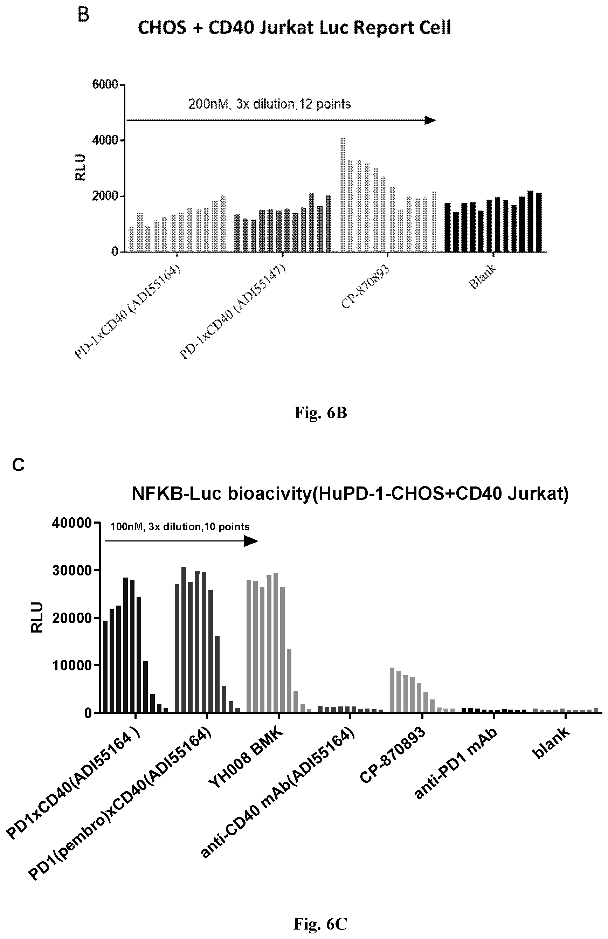

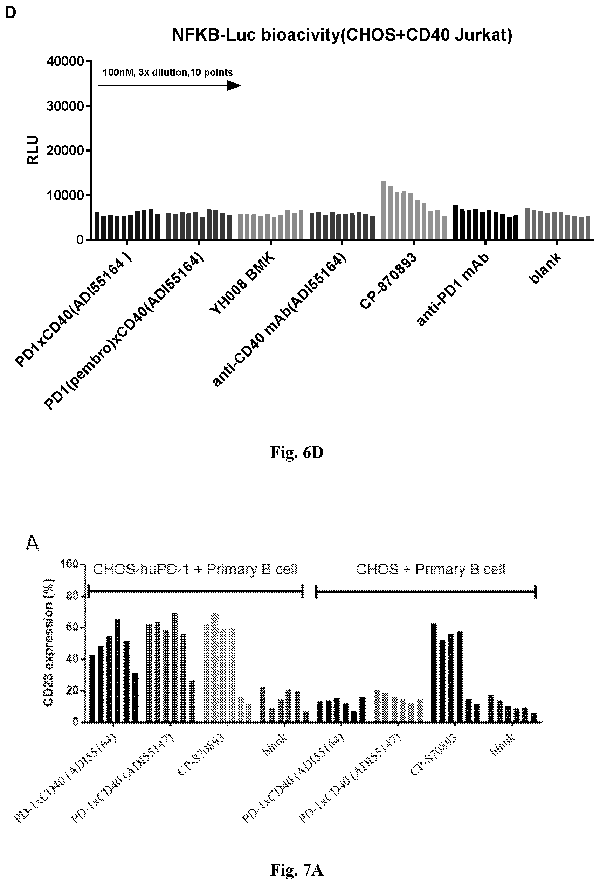

Results are shown in Figs. 6A-6D. The results show that the bispecific antibody samples did not show significant signal activation in the co-incubation system of huCD40 Jurkat-NF-κB cells and CHO-S cells (without PD-1 expression), but showed significant signal activation in the co-incubation system of huCD40 Jurkat-NF-κB cells and CHO-S cells overexpressing human PD-1. This is because the aggregation effect of the antibody induced by PD-1 also brings the CD40 on the Jurkat cells bound by the other end of the antibody together, activating the NF-κB pathway.

### Example 7: Detection of Activation Activity of Bispecific Antibodies (Primary B Cell Activation)

CHO-S cells overexpressing human PD-1 or CHO-S cells not expressing PD-1 were treated with mitomycin C for 4 hours, and cell density was adjusted to 2 × 10⁶ cells/ml with X-VIVO15 medium. Frozen human PBMCs (purchased from Shanghai Saily) were resuscitated, and B cells were isolated according to the instructions of the EasySep human B cell enrichment kit. B cells were adjusted to a density of 1 × 10⁶ cells/mL with X-ViVOTM15 medium. HuPD-1-CHOS/HuPD-L1-CHOS and CHOS cells (treated with mitomycin C and washed three times) were collected and adjusted to a density of 1 × 10⁶ cells/mL with X-ViVOTM15 medium. Antibodies were diluted in X-ViVOTM15 medium, starting from 200 nM with 3-fold serial dilution. 50 µL/well of antibody dilution was added to the plate, followed by 50 µL/well of target cells, and incubated for 30 min. 50 µL/well of B cells were then added and incubated for 48 h before detecting CD23 and CD86 expression.

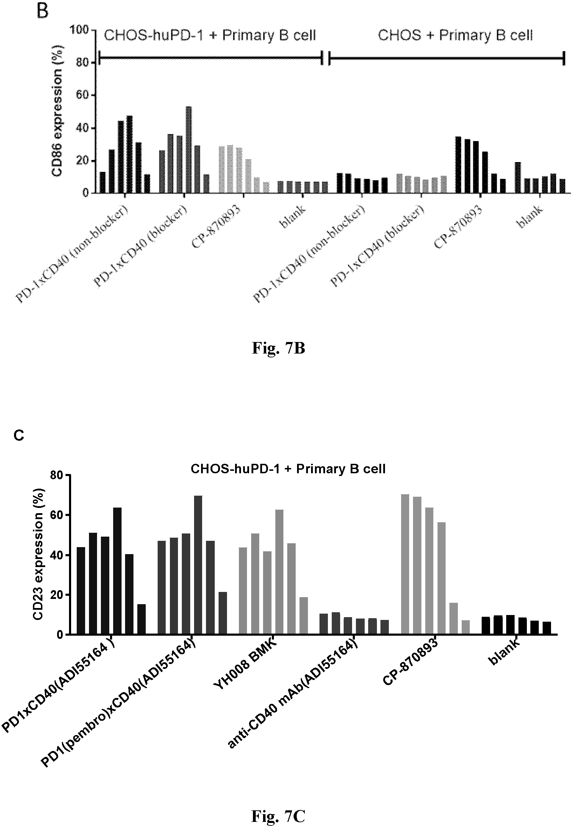

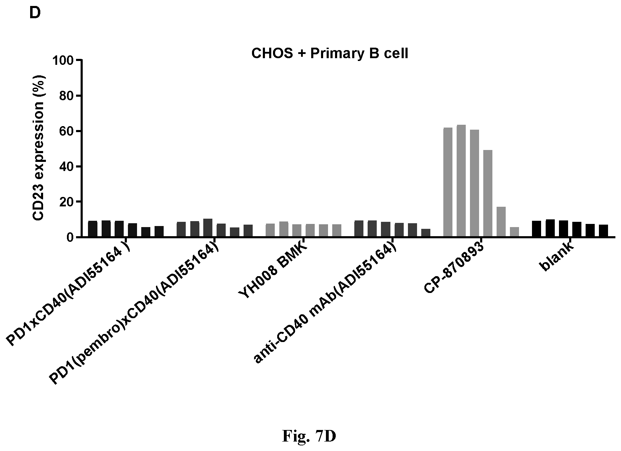

The experimental results are shown in Figs. 7A-7D. No significant signal activation by the bispecific antibody samples was observed in the co-incubation system of B cells and CHO-S cells not expressing PD-1, but significant B cell activation was observed in the co-incubation system of B cells and CHO-S cells overexpressing human PD-1.

### Example 8: Mixed Lymphocyte Reaction with Bispecific Antibodies

PBMC cells (purchased from SAILY BIO, SLB-HPB) were resuscitated, centrifuged, and resuspended in 10 ml X-VIVO-15 medium (purchased from LONZA). PBMCs were allowed to adhere in a cell culture incubator at 37°C for 2 hours, and non-adherent cells were removed. 10 ml of DC medium: X-VIVO-15 medium supplemented with 10 ng/ml GM-CSF (purchased from R&D) and 20 ng/ml IL-4 (purchased from R&D) was added, and the cells were cultured for 3 days. An additional 5 ml of DC medium was added, and the culture was continued until day 6. DC maturation medium: X-VIVO-15 medium supplemented with 1000 U/ml TNF-α (purchased from R&D), 10 ng/ml IL-6 (purchased from R&D), 5 ng/ml IL-1β (purchased from R&D), and 1 µM PGE2 (purchased from Tocris) was added, and the cells were cultured for 2 days. Mature DC cells were collected and adjusted to a cell density of 2 × 10⁵ cells/ml with X-VIVO-15 medium. PBMC cells from another donor (purchased from SAILY BIO, SLB-HPB) were resuscitated, centrifuged, and resuspended in 10 ml X-VIVO-15 medium. T cells were enriched using a T Cell Isolation Kit (purchased from Stemcell), resuspended in X-VIVO-15 medium, and adjusted to a cell density of 2 × 10⁶ cells/ml. The T cells were mixed with the previously collected mature DC cells at a 1:1 ratio. 100 µL/well of the mixture was added to a 96-well U-bottom plate, and 100 µL/well of bispecific antibody samples diluted in X-VIVO-15 medium were added simultaneously. After 3 days of culture, supernatants were collected, and IL-2 expression was detected by ELISA (purchased from eBioscience).

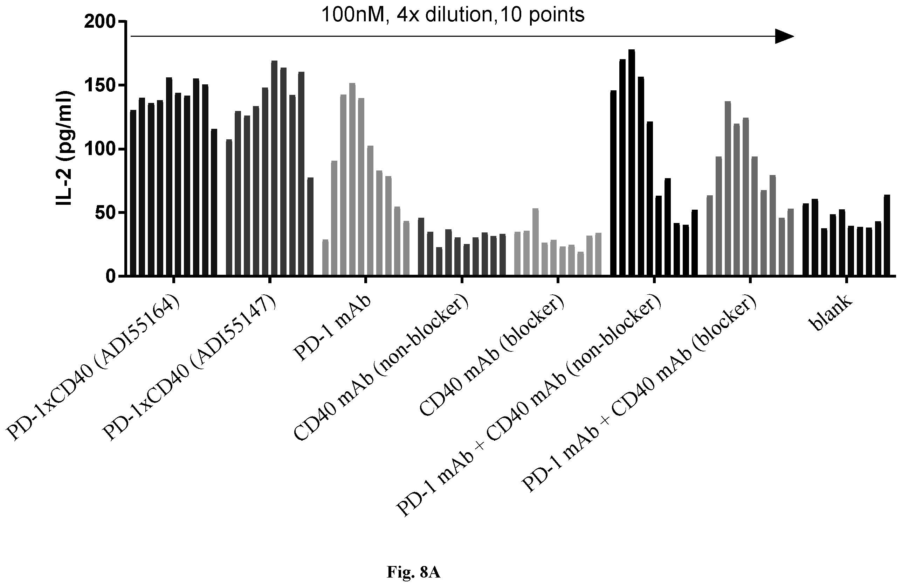

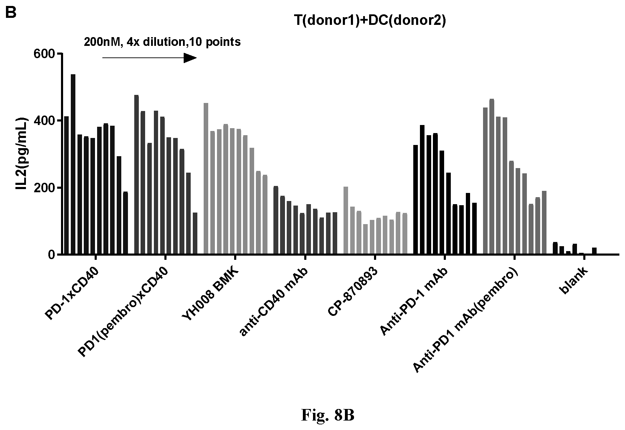

The experimental results are shown in Figs. 8A-8B. The bispecific antibody samples can activate T cells to release IL-2 in the mixed lymphocyte reaction system.

### Example 9: Half-Life of Bispecific Antibodies in Mice

Balb/c mice, half male and half female, were used in the experiment, maintained on a 12/12-hour light/dark cycle, at a temperature of 24 ± 2°C and humidity of 40-70%, with *ad libitum* access to water and food. On the day of the experiment, Balb/c mice were dosed via tail vein with a single injection of bispecific antibody molecules at a dose of 10 mg/kg. Blood was collected from the mouse orbital sinus at time points: 5 minutes, 0.5 hour, 2 hours, 6 hours, 24 hours, 48 hours, 96 hours, 168 hours, 336 hours, and 504 hours post-dosing. Whole blood samples were placed at 2-8°C for 30 minutes, centrifuged at 12000 rpm for 5 minutes to collect serum. The obtained serum was centrifuged again at 2-8°C, 12000 rpm for 5 minutes, and stored at -80°C. The content of bispecific antibody molecules in the serum was detected by ELISA.

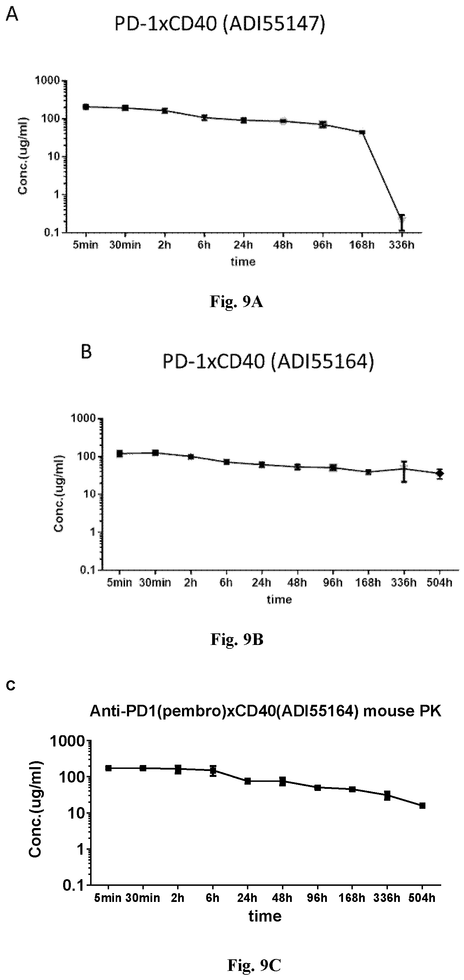

Results are shown in Figs. 9A-9C. The results show that the half-lives of the bispecific antibody molecules in mice were approximately 133 hours (PD-1xCD40(blocker), Fig. 9A), 238 hours (PD-1xCD40(non-blocker), Fig. 9B), and 222 hours (PD-1(pembro)xCD40(non-blocker), Fig. 9C), respectively.

### Example 10: In Vivo Pharmacodynamic Study of Bispecific Antibodies

### 1. In Vivo Pharmacodynamic Study of Bispecific Antibodies in a B-NDG B2M KO Plus Mouse Tumor Model with Subcutaneous Mixed Inoculation of A375 and Human PBMCs

This experiment determined the anti-tumor activity of the bispecific antibodies of the present disclosure in a B-NDG B2M KO Plus mouse tumor model with subcutaneous mixed inoculation of A375 and human PBMCs. Specifically, an A375 tumor-bearing mouse model was first established by subcutaneous mixed inoculation of A375 + PBMCs. When the average tumor volume reached approximately 300 mm³, mice were grouped and administered intraperitoneally with PBS, 7 mg/kg anti-CD40 monoclonal antibody, 7 mg/kg anti-PD-1 monoclonal antibody, 7 mg/kg anti-PD-1 monoclonal antibody combined with 7 mg/kg anti-CD40 monoclonal antibody, or 9.6 mg/kg anti-PD-1/CD40 bispecific antibody, respectively. Changes in tumor volume and body weight of mice in each group were monitored every 2-3 days for a total of 5 consecutive measurements. The dosage and regimen are shown in Table 3.

**Table 3: Dosing Regimen for In Vivo Pharmacodynamic Experiment**

| Group | Dosage | Dosing frequency/times |
|---|---|---|
| PBS | N/A | Q2-3D*4 times |
| Anti-CD40 mAb (ADI-55164) | 7mg/kg | Q2-3D*4 times |
| Anti-PD-1 mAb(ADI-54872) | 7mg/kg | Q2-3D*4 times |
| Anti-PD-1 mAb (ADI-54872)+ Anti-CD40 mAb (ADI-55164) | 7mg/kg+7mg/kg | Q2-3D*4 times |
| Anti-PD-1/CD40 bispecific Ab (PD-1×CD40 (ADI-55164)) | 9.6mg/kg | Q2-3D*4 times |

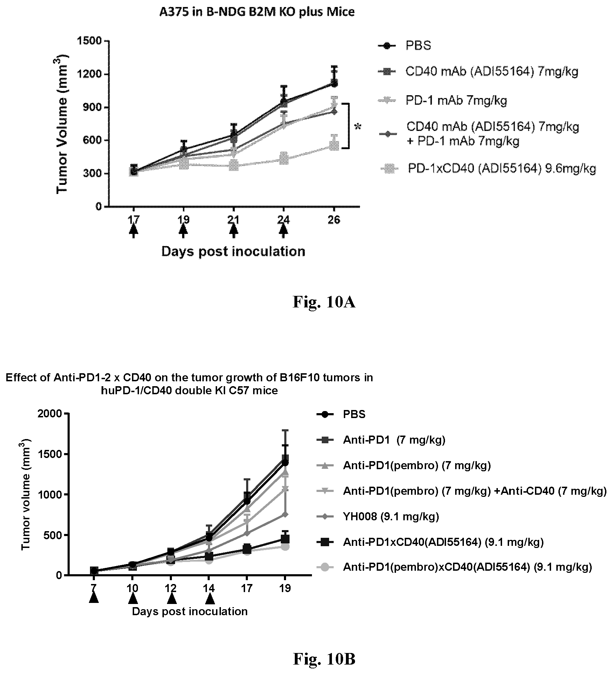

Results are shown in Fig. 10A. The anti-PD-1/CD40 bispecific antibody of the present disclosure exhibits significant anti-tumor activity, which is significantly superior to the anti-PD-1 monoclonal antibody and the combination regimen of anti-PD-1 monoclonal antibody and anti-CD40 monoclonal antibody.

### 2. In Vivo Pharmacodynamic Study of Bispecific Antibodies in a huPD-1/CD40 Double KI C57 Mouse Tumor Model with Subcutaneous Mixed Inoculation of B16F10 and Human PBMCs

First, a B16F10 tumor-bearing mouse model was established by subcutaneous inoculation of B16F10. After approximately seven days, mice were grouped and intraperitoneally injected with PBS, 7 mg/kg anti-PD-1 monoclonal antibody, 7 mg/kg anti-PD-1 monoclonal antibody in combination with 7 mg/kg anti-CD40 monoclonal antibody, 9.1 mg/kg YH008 BMK, and the anti-PD-1/CD40 bispecific antibodies PD1xCD40(ADI55164) and PD1(Pembro)xCD40(ADI55164), respectively. Changes in tumor volume and body weight of mice in each group were monitored continuously every 2- 3 days. The dosage and regimen are shown in Table 4.

**Table 4: Dosing Regimen for In Vivo Pharmacodynamic Experiment**

| Group | Dosage | Dosing frequency/times |
|---|---|---|
| PBS | N/A | Q2-3D*4 times |
| Anti-PD1(ADI54872) | 7mg/kg | Q2-3D*4 times |
| Pembrolizumab-G1 | 7mg/kg | Q2-3D*4 times |
| Pembrolizumab-G1+Anti-CD40-G1(ADI55164) | 7mg/kg+7mg/kg | Q2-3D*4 times |
| YH008 BMK | 9.1mg/kg | Q2-3D*4 times |
| PD1xCD40(ADI55164) | 9.1mg/kg | Q2-3D*4 times |
| PD1(Pembro)xCD40(ADI55164) | 9.1mg/kg | Q2-3D*4 times |

Results are shown in Fig. 10B. The bispecific antibodies PD1xCD40(ADI55164) and PD1(Pembro)xCD40(ADI55164) of the present disclosure exhibit significant anti-tumor activity, which is significantly superior to the anti-PD-1 monoclonal antibody, the combination of anti-PD-1 monoclonal antibody and anti-CD40 monoclonal antibody, and YH008 BMK.

Although the specific embodiments of the present disclosure have been described in detail, those skilled in the art will appreciate that various modifications and changes may be made to the details based on all the teachings disclosed, and such changes are within the protection scope of the present disclosure. The entire scope of the present disclosure is given by the appended claims and any equivalents thereof.

## Claims

1. A bispecific antibody comprising a first antigen-binding domain that specifically binds to PD-1 and a second antigen-binding domain that specifically binds to CD40;
wherein the first antigen-binding domain comprises a first light chain variable region (VL) and a first heavy chain variable region (VH) which together form a domain capable of specifically binding to PD-1; and the second antigen-binding domain comprises a second light chain variable region (VL) and a second heavy chain variable region (VH) which together form a domain capable of specifically binding to CD40.

2. The bispecific antibody of claim 1, wherein the first antigen-binding domain and the second antigen-binding domain are each independently an scFv or a Fab.

3. The bispecific antibody of claim 1 or 2, wherein:
(a) the first light chain variable region (VL) comprises a LCDR1, a LCDR2, and a LCDR3 comprised in the VL as set forth in SEQ ID NO: 4; and/or the first heavy chain variable region (VH) comprises a HCDR1, a HCDR2, and a HCDR3 comprised in the VH as set forth in SEQ ID NO: 3; or
(b) the first light chain variable region (VL) comprises a LCDR1, a LCDR2, and a LCDR3 comprised in the VL as set forth in SEQ ID NO: 40; and/or the first heavy chain variable region (VH) comprises a HCDR1, a HCDR2, and a HCDR3 comprised in the VH as set forth in SEQ ID NO: 39.

4. The bispecific antibody of any one of claims 1-3, wherein:
(a) the first light chain variable region (VL) comprises a LCDR1 as set forth in SEQ ID NO: 16, a LCDR2 as set forth in SEQ ID NO: 17, and a LCDR3 as set forth in SEQ ID NO: 18; and/or the first heavy chain variable region (VH) comprises a HCDR1 as set forth in SEQ ID NO: 13, a HCDR2 as set forth in SEQ ID NO: 14, and a HCDR3 as set forth in SEQ ID NO: 15; or
(b) the first light chain variable region (VL) comprises a LCDR1 as set forth in SEQ ID NO: 44, a LCDR2 as set forth in SEQ ID NO: 45, and a LCDR3 as set forth in SEQ ID NO: 46; and/or the first heavy chain variable region (VH) comprises a HCDR1 as set forth in SEQ ID NO: 41, a HCDR2 as set forth in SEQ ID NO: 42, and a HCDR3 as set forth in SEQ ID NO: 43;
preferably, the CDRs are defined by the IMGT numbering system.

5. The bispecific antibody of any one of claims 1-4, wherein:
(a) the first light chain variable region (VL) comprises an amino acid sequence as set forth in SEQ ID NO: 4 or a variant thereof, and/or the first heavy chain variable region (VH) comprises an amino acid sequence as set forth in SEQ ID NO: 3 or a variant thereof; or
(b) the first light chain variable region (VL) comprises an amino acid sequence as set forth in SEQ ID NO: 40 or a variant thereof, and/or the first heavy chain variable region (VH) comprises an amino acid sequence as set forth in SEQ ID NO: 39 or a variant thereof;
wherein the variant has one or several amino acid substitutions, deletions, or additions (e.g., 1, 2, 3, 4, or 5 amino acid substitutions, deletions, or additions) compared to the sequence from which it is derived, or has a sequence having at least 80%, at least 85%, at least 90%, at least 91%, at least 92%, at least 93%, at least 94%, at least 95%, at least 96%, at least 97%, at least 98%, at least 99%, or 100% sequence identity thereto; preferably, the substitutions are conservative substitutions.

6. The bispecific antibody of any one of claims 1-5, wherein:
(a) the second light chain variable region (VL) comprises a LCDR1, a LCDR2, and a LCDR3 comprised in the VL as set forth in SEQ ID NO: 23 or 33; and/or the second heavy chain variable region (VH) comprises a HCDR1, a HCDR2, and a HCDR3 comprised in the VH as set forth in SEQ ID NO: 5 or 31; or
(b) the second light chain variable region (VL) comprises a LCDR1, a LCDR2, and a LCDR3 comprised in the VL as set forth in SEQ ID NO: 24 or 34; and/or the second heavy chain variable region (VH) comprises a HCDR1, a HCDR2, and a HCDR3 comprised in the VH as set forth in SEQ ID NO: 6 or 32.

7. The bispecific antibody of any one of claims 1-6, wherein:
(a) the second light chain variable region (VL) comprises a LCDR1 as set forth in SEQ ID NO: 25, a LCDR2 as set forth in SEQ ID NO: 26, and a LCDR3 as set forth in SEQ ID NO: 27; and/or the second heavy chain variable region (VH) comprises a HCDR1 comprising the amino acid sequence as set forth in SEQ ID NO: 7, a HCDR2 comprising the amino acid sequence as set forth in SEQ ID NO: 8, and a HCDR3 comprising the amino acid sequence as set forth in SEQ ID NO: 9;
or
(b) the second light chain variable region (VL) comprises a LCDR1 as set forth in SEQ ID NO: 28, a LCDR2 as set forth in SEQ ID NO: 29, and a LCDR3 as set forth in SEQ ID NO: 30; and/or the second heavy chain variable region (VH) comprises a HCDR1 comprising the amino acid sequence as set forth in SEQ ID NO: 10, a HCDR2 comprising the amino acid sequence as set forth in SEQ ID NO: 11, and a HCDR3 comprising the amino acid sequence as set forth in SEQ ID NO: 12;
preferably, the CDRs are defined by the IMGT numbering system.

8. The bispecific antibody of any one of claims 1-7, wherein:
(a) the second light chain variable region (VL) comprises an amino acid sequence as set forth in SEQ ID NO: 23 or 33 or a variant thereof, and/or the second heavy chain variable region (VH) comprises an amino acid sequence as set forth in SEQ ID NO: 5 or 31 or a variant thereof;
or
(b) the second light chain variable region (VL) comprises an amino acid sequence as set forth in SEQ ID NO: 24 or 34 or a variant thereof, and/or the second heavy chain variable region (VH) comprises an amino acid sequence as set forth in SEQ ID NO: 6 or 32 or a variant thereof;
wherein the variant has one or several amino acid substitutions, deletions, or additions (e.g., 1, 2, 3, 4, or 5 amino acid substitutions, deletions, or additions) compared to the sequence from which it is derived, or has a sequence having at least 80%, at least 85%, at least 90%, at least 91%, at least 92%, at least 93%, at least 94%, at least 95%, at least 96%, at least 97%, at least 98%, at least 99%, or 100% sequence identity thereto; preferably, the substitutions are conservative substitutions;
e.g., the second light chain variable region (VL) comprises an amino acid sequence as set forth in SEQ ID NO: 23, and/or the second heavy chain variable region (VH) comprises an amino acid sequence as set forth in SEQ ID NO: 5;
e.g., the second light chain variable region (VL) comprises an amino acid sequence as set forth in SEQ ID NO: 33, and/or the second heavy chain variable region (VH) comprises an amino acid sequence as set forth in SEQ ID NO: 31;
e.g., the second light chain variable region (VL) comprises an amino acid sequence as set forth in SEQ ID NO: 24, and/or the second heavy chain variable region (VH) comprises an amino acid sequence as set forth in SEQ ID NO: 6;
e.g., the second light chain variable region (VL) comprises an amino acid sequence as set forth in SEQ ID NO: 34, and/or the second heavy chain variable region (VH) comprises an amino acid sequence as set forth in SEQ ID NO: 32.

9. The bispecific antibody of any one of claims 1-8, comprising:
(1) the first light chain variable region (VL) comprising a LCDR1, a LCDR2, and a LCDR3 comprised in the VL as set forth in SEQ ID NO: 4, the first heavy chain variable region (VH) comprising a HCDR1, a HCDR2, and a HCDR3 comprised in the VH as set forth in SEQ ID NO: 3, the second light chain variable region (VL) comprising a LCDR1, a LCDR2, and a LCDR3 comprised in the VL as set forth in SEQ ID NO: 23 or 33, and the second heavy chain variable region (VH) comprising a HCDR1, a HCDR2, and a HCDR3 comprised in the VH as set forth in SEQ ID NO: 5 or 31;
(2) the first light chain variable region (VL) comprising a LCDR1, a LCDR2, and a LCDR3 comprised in the VL as set forth in SEQ ID NO: 4, the first heavy chain variable region (VH) comprising a HCDR1, a HCDR2, and a HCDR3 comprised in the VH as set forth in SEQ ID NO: 3, the second light chain variable region (VL) comprising a LCDR1, a LCDR2, and a LCDR3 comprised in the VL as set forth in SEQ ID NO: 24 or 34, and the second heavy chain variable region (VH) comprising a HCDR1, a HCDR2, and a HCDR3 comprised in the VH as set forth in SEQ ID NO: 6 or 32;
(3) the first light chain variable region (VL) comprising a LCDR1, a LCDR2, and a LCDR3 comprised in the VL as set forth in SEQ ID NO: 40, the first heavy chain variable region (VH) comprising a HCDR1, a HCDR2, and a HCDR3 comprised in the VH as set forth in SEQ ID NO: 39, the second light chain variable region (VL) comprising a LCDR1, a LCDR2, and a LCDR3 comprised in the VL as set forth in SEQ ID NO: 23 or 33, and the second heavy chain variable region (VH) comprising a HCDR1, a HCDR2, and a HCDR3 comprised in the VH as set forth in SEQ ID NO: 5 or 31; or
(4) the first light chain variable region (VL) comprising a LCDR1, a LCDR2, and a LCDR3 comprised in the VL as set forth in SEQ ID NO: 40, the first heavy chain variable region (VH) comprising a HCDR1, a HCDR2, and a HCDR3 comprised in the VH as set forth in SEQ ID NO: 39, the second light chain variable region (VL) comprising a LCDR1, a LCDR2, and a LCDR3 comprised in the VL as set forth in SEQ ID NO: 24 or 34, and the second heavy chain variable region (VH) comprising a HCDR1, a HCDR2, and a HCDR3 comprised in the VH as set forth in SEQ ID NO: 6 or 32.

10. The bispecific antibody of any one of claims 1-9, comprising:
(1) the first light chain variable region (VL) comprising a LCDR1 as set forth in SEQ ID NO: 16, a LCDR2 as set forth in SEQ ID NO: 17, and a LCDR3 as set forth in SEQ ID NO: 18, the first heavy chain variable region (VH) comprising a HCDR1 as set forth in SEQ ID NO: 13, a HCDR2 as set forth in SEQ ID NO: 14, and a HCDR3 as set forth in SEQ ID NO: 15; the second light chain variable region (VL) comprising a LCDR1 as set forth in SEQ ID NO: 25, a LCDR2 as set forth in SEQ ID NO: 26, and a LCDR3 as set forth in SEQ ID NO: 27, and the second heavy chain variable region (VH) comprising a HCDR1 as set forth in the amino acid sequence of SEQ ID NO: 7, a HCDR2 as set forth in the amino acid sequence of SEQ ID NO: 8, and a HCDR3 as set forth in the amino acid sequence of SEQ ID NO: 9;
(2) the first light chain variable region (VL) comprising a LCDR1 as set forth in SEQ ID NO: 16, a LCDR2 as set forth in SEQ ID NO: 17, and a LCDR3 as set forth in SEQ ID NO: 18, the first heavy chain variable region (VH) comprising a HCDR1 as set forth in SEQ ID NO: 13, a HCDR2 as set forth in SEQ ID NO: 14, and a HCDR3 as set forth in SEQ ID NO: 15; the second light chain variable region (VL) comprising a LCDR1 as set forth in SEQ ID NO: 28, a LCDR2 as set forth in SEQ ID NO: 29, and a LCDR3 as set forth in SEQ ID NO: 30, and the second heavy chain variable region (VH) comprising a HCDR1 as set forth in the amino acid sequence of SEQ ID NO: 10, a HCDR2 as set forth in the amino acid sequence of SEQ ID NO: 11, and a HCDR3 as set forth in the amino acid sequence of SEQ ID NO: 12;
(3) the first light chain variable region (VL) comprising a LCDR1 as set forth in SEQ ID NO: 44, a LCDR2 as set forth in SEQ ID NO: 45, and a LCDR3 as set forth in SEQ ID NO: 46, the first heavy chain variable region (VH) comprising a HCDR1 as set forth in SEQ ID NO: 41, a HCDR2 as set forth in SEQ ID NO: 42, and a HCDR3 as set forth in SEQ ID NO: 43, the second light chain variable region (VL) comprising a LCDR1 as set forth in SEQ ID NO: 25, a LCDR2 as set forth in SEQ ID NO: 26, and a LCDR3 as set forth in SEQ ID NO: 27, and the second heavy chain variable region (VH) comprising a HCDR1 as set forth in the amino acid sequence of SEQ ID NO: 7, a HCDR2 as set forth in the amino acid sequence of SEQ ID NO: 8, and a HCDR3 as set forth in the amino acid sequence of SEQ ID NO: 9; or
(4) the first light chain variable region (VL) comprising a LCDR1 as set forth in SEQ ID NO: 44, a LCDR2 as set forth in SEQ ID NO: 45, and a LCDR3 as set forth in SEQ ID NO: 46, the first heavy chain variable region (VH) comprising a HCDR1 as set forth in SEQ ID NO: 41, a HCDR2 as set forth in SEQ ID NO: 42, and a HCDR3 as set forth in SEQ ID NO: 43, the second light chain variable region (VL) comprising a LCDR1 as set forth in SEQ ID NO: 28, a LCDR2 as set forth in SEQ ID NO: 29, and a LCDR3 as set forth in SEQ ID NO: 30, and the second heavy chain variable region (VH) comprising a HCDR1 as set forth in the amino acid sequence of SEQ ID NO: 10, a HCDR2 as set forth in the amino acid sequence of SEQ ID NO: 11, and a HCDR3 as set forth in the amino acid sequence of SEQ ID NO: 12;
preferably, wherein the bispecific antibody comprises:
(1) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 23, and the second heavy chain variable region (VH) comprising the amino acid sequence as set forth in SEQ ID NO: 5;
(2) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 33, and the second heavy chain variable region (VH) comprising the amino acid sequence as set forth in SEQ ID NO: 31;
(3) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 24, and the second heavy chain variable region (VH) comprising the amino acid sequence as set forth in SEQ ID NO: 6;
(4) the first light chain variable region (VL) comprising the amino acid sequence as set forth in SEQ ID NO: 4, the first heavy chain variable region (VH) comprising the amino acid sequence as set forth in SEQ ID NO: 3, the second light chain variable region (VL) comprising the amino acid sequence as set forth in SEQ ID NO: 34, and the second heavy chain variable region (VH) comprising the amino acid sequence as set forth in SEQ ID NO: 32;
(5) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 23, and the second heavy chain variable region (VH) comprising the amino acid sequence as set forth in SEQ ID NO: 5;
(6) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 33, and the second heavy chain variable region (VH) comprising the amino acid sequence as set forth in SEQ ID NO: 31;
(7) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 24, and the second heavy chain variable region (VH) comprising the amino acid sequence as set forth in SEQ ID NO: 6; or
(8) the first light chain variable region (VL) comprising the amino acid sequence as set forth in SEQ ID NO: 40, the first heavy chain variable region (VH) comprising the amino acid sequence as set forth in SEQ ID NO: 39, the second light chain variable region (VL) comprising the amino acid sequence as set forth in SEQ ID NO: 34, and the second heavy chain variable region (VH) comprising the amino acid sequence as set forth in SEQ ID NO: 32.

11. The bispecific antibody of any one of claims 1-10, wherein the first antigen-binding domain is a Fab and the second antigen-binding domain is an scFv.

12. The bispecific antibody of claim 11, wherein the bispecific antibody comprises a peptide chain I and a peptide chain II; wherein the peptide chain I comprises the first light chain variable region and a light chain constant region, and the peptide chain II comprises: the first heavy chain variable region, a heavy chain constant region, the second heavy chain variable region, and the second light chain variable region.

13. The bispecific antibody of claim 12, wherein the peptide chain I comprises, from N-terminus to C-terminus, the first light chain variable region and the light chain constant region, and/or the peptide chain II comprises, from N-terminus to C-terminus: (i) the first heavy chain variable region, the heavy chain constant region, the second heavy chain variable region, and the second light chain variable region; or (ii) the first heavy chain variable region, the heavy chain constant region, the second light chain variable region, and the second heavy chain variable region.

14. The bispecific antibody of claim 12 or 13, wherein adjacent domains of the peptide chain I are optionally connected via or without a linker, and/or adjacent domains of the peptide chain II are optionally connected via or without a linker;
preferably, the linker is each independently the same or a different peptide linker (e.g., a rigid peptide linker or a flexible peptide linker); preferably, the peptide linker is each independently selected from a peptide linker comprising one or more glycines (G) and/or serines (S) and/or alanines (A), e.g., having the structure represented by (GGGGS)n or (GGGGA)n, wherein n is selected from 1, 2, 3, 4, 5, 6, 7, 8, 9, or 10; preferably, the peptide linker comprises an amino acid sequence as set forth in SEQ ID NO: 36 or 37;
preferably, the peptide chain II comprises, from N-terminus to C-terminus, the first heavy chain variable region, the heavy chain constant region, the second heavy chain variable region, and the second light chain variable region, wherein the heavy chain constant region and the second heavy chain variable region are connected via a peptide linker comprising one or more glycines (G) and/or serines (S) (e.g., a peptide linker as set forth in SEQ ID NO: 36); or the peptide chain II comprises, from N-terminus to C-terminus, the first heavy chain variable region, the heavy chain constant region, the second heavy chain variable region, and the second light chain variable region, wherein the heavy chain constant region and the second heavy chain variable region are connected via a peptide linker comprising one or more glycines (G) and/or alanines (A) (e.g., a peptide linker as set forth in SEQ ID NO: 36);
and/or
the second heavy chain variable region and the second light chain variable region are connected via a peptide linker comprising one or more glycines (G) and/or serines (S) (e.g., a peptide linker as set forth in SEQ ID NO: 37).

15. The bispecific antibody of any one of claims 11-14, wherein an intrachain disulfide
bond can be formed between the second light chain variable region and the second heavy chain variable region;
preferably, (a) the second light chain variable region comprises the amino acid sequence as set forth in SEQ ID NO: 33, and the second heavy chain variable region comprises the amino acid sequence as set forth in SEQ ID NO: 31; or (b) the second light chain variable region comprises the amino acid sequence as set forth in SEQ ID NO: 34, and the second heavy chain variable region comprises the amino acid sequence as set forth in SEQ ID NO: 32.

16. The bispecific antibody of any one of claims 12-15, wherein the heavy chain
constant region is derived from a human immunoglobulin (e.g., IgG1, IgG2, IgG3, or IgG4);
preferably, the heavy chain constant region is selected from a heavy chain constant region of a wild-type human immunoglobulin (e.g., IgG1, IgG2, IgG3, or IgG4) or a variant thereof (e.g., a heavy chain constant region comprising a mutation or a chemical modification); wherein the Fc domain comprised in the heavy chain constant region variant has an altered (e.g., enhanced or reduced) effector functions compared to the heavy chain constant region of the wild-type immunoglobulin from which it is derived; e.g., the Fc domain comprised in the heavy chain constant region variant has a reduced ADCC, ADCP, and/or CDC activity compared to the heavy chain constant region of the wild-type immunoglobulin from which it is derived;
preferably, the heavy chain constant region is selected from a heavy chain constant region of a wild-type human immunoglobulin IgG1 or a variant thereof (e.g., a heavy chain constant region comprising a mutation or a chemical modification); wherein the heavy chain constant region variant comprises L234A, L235A, and/or L237A substitution mutations compared to the heavy chain constant region of the wild-type immunoglobulin from which it is derived;
preferably, the Fc domain monomer comprises an amino acid sequence as set forth in SEQ ID NO: 19.

17. The bispecific antibody of any one of claims 12-16, wherein the light chain
constant region is derived from a kappa (κ) or lambda (λ) light chain of a human immunoglobulin;
preferably, the light chain constant region comprises an amino acid sequence as set forth in SEQ ID NO: 20.

18. The bispecific antibody of any one of claims 12-17, wherein:
(a) the peptide chain I comprises an amino acid sequence as set forth in SEQ ID NO: 35 or a variant thereof, and/or the peptide chain II comprises an amino acid sequence as set forth in SEQ ID NO: 1 or 2 or a variant thereof; or
(b) the peptide chain I comprises an amino acid sequence as set forth in SEQ ID NO: 47 or a variant thereof, and/or the peptide chain II comprises an amino acid sequence as set forth in SEQ ID NO: 38 or a variant thereof;
wherein the variant has one or several amino acid substitutions, deletions, or additions (e.g., 1, 2, 3, 4, or 5 amino acid substitutions, deletions, or additions) compared to the sequence from which it is derived, or has a sequence having at least 80%, at least 85%, at least 90%, at least 91%, at least 92%, at least 93%, at least 94%, at least 95%, at least 96%, at least 97%, at least 98%, at least 99%, or 100% sequence identity thereto; preferably, the substitutions are conservative substitutions;
preferably, the bispecific antibody comprises:
(1) the peptide chain I comprising the amino acid sequence as set forth in SEQ ID NO: 35, and the peptide chain II comprising the amino acid sequence as set forth in SEQ ID NO: 1;
(2) the peptide chain I comprising the amino acid sequence as set forth in SEQ ID NO: 35, and the peptide chain II comprising the amino acid sequence as set forth in SEQ ID NO: 2; or
(3) the peptide chain I comprising the amino acid sequence as set forth in SEQ ID NO: 47, and the peptide chain II comprising the amino acid sequence as set forth in SEQ ID NO: 38.

19. The bispecific antibody of any one of claims 12-18, comprising two peptide chains I and two peptide chains II;
preferably, the two peptide chains I comprised in the bispecific antibody are identical or different, and/or the two peptide chains II comprised in the bispecific antibody are identical or different;
preferably, the bispecific antibody comprises two identical peptide chains I and two identical peptide chains II.

20. An isolated nucleic acid molecule or set of nucleic acid molecules comprising a nucleotide sequence encoding the bispecific antibody of any one of claims 1-19.

21. A vector comprising the isolated nucleic acid molecule or set of nucleic acid molecules of claim 20;
preferably, the nucleotide sequences encoding different peptide chains of the bispecific antibody are located on different vector molecules;
preferably, the vector is a cloning vector or an expression vector.

22. A host cell comprising the isolated nucleic acid molecule or set of nucleic acid molecules of claim 20, or the vector of claim 21.

23. A method for preparing the bispecific antibody of any one of claims 1-19, comprising culturing the host cell of claim 22 under conditions allowing expression of the bispecific antibody, and recovering the bispecific antibody from the cultured host cell culture.

24. A pharmaceutical composition comprising the bispecific antibody of any one of claims 1-19, or the isolated nucleic acid molecule or set of nucleic acid molecules of claim 20, or the vector of claim 21, or the host cell of claim 22, and a pharmaceutically acceptable carrier and/or excipient.

25. The pharmaceutical composition of claim 24, wherein the pharmaceutical composition further comprises an additional pharmaceutically active agent;
preferably, the additional pharmaceutically active agent is a drug having anti-tumor activity;
preferably, the bispecific antibody and the additional pharmaceutically active agent are provided as separate components or as a mixed component.

26. Use of the bispecific antibody of any one of claims 1-19, or the isolated nucleic acid molecule or set of nucleic acid molecules of claim 20, or the vector of claim 21, or the host cell of claim 22, or the pharmaceutical composition of claim 24 or 25 for the manufacture of a medicament for use in:
(1) enhancing immune cell activity in vitro or in a subject (e.g., a human or a monkey);
(2) enhancing an immune response in a subject (e.g., a human or a monkey);
(3) preventing and/or treating a tumor in a subject (e.g., a human or a monkey); and/or
(4) preventing and/or treating an infection in a subject (e.g., a human or a monkey); preferably, the immune cell is a T cell, a B cell, a DC cell, a macrophage, and/or a NK cell;
preferably, the tumor is a solid tumor, such as melanoma (e.g., metastatic malignant melanoma), breast cancer, renal cancer (e.g., clear cell carcinoma), prostate cancer, bladder cancer, pancreatic cancer, lung cancer (e.g., non-small cell lung cancer), colon cancer, esophageal cancer, head and neck squamous cell carcinoma, liver cancer, ovarian cancer, cervical cancer, thyroid cancer, glioblastoma, or glioma;
preferably, the tumor is a hematologic malignancy, such as lymphoma, leukemia; preferably, the lymphoma is Hodgkin's lymphoma or non-Hodgkin's lymphoma; preferably, the non-Hodgkin's lymphoma is one or more of peripheral T-cell lymphoma, angioimmunoblastic T-cell lymphoma, EBV-positive NK/T-cell lymphoma (nasal type), and B-cell non-Hodgkin's lymphoma;
preferably, the infection is selected from a viral infection, a bacterial infection, a fungal infection, and a parasitic infection;
preferably, the subject is a mammal, e.g., a human or a monkey.

27. The use of claim 26, wherein the bispecific antibody, the isolated nucleic acid
molecule or set of nucleic acid molecules, the vector, the host cell, the conjugate, or the pharmaceutical composition is administered in combination with an additional pharmaceutically active agent, e.g., simultaneously, separately, or sequentially;
preferably, the additional pharmaceutically active agent is a drug having anti-tumor activity.

28. A method for enhancing an immune response and/or preventing and/or treating a tumor or an infection in a subject; the method comprising: administering an effective amount of the bispecific antibody of any one of claims 1-19, or the isolated nucleic acid molecule or set of nucleic acid molecules of claim 20, or the vector of claim 21, or the host cell of claim 22, or the pharmaceutical composition of claim 24 or 25 to a subject in need thereof;
preferably, the tumor is a solid tumor, such as melanoma (e.g., metastatic malignant melanoma), breast cancer, renal cancer (e.g., clear cell carcinoma), prostate cancer, bladder cancer, pancreatic cancer, lung cancer (e.g., non-small cell lung cancer), colon cancer, esophageal cancer, head and neck squamous cell carcinoma, liver cancer, ovarian cancer, cervical cancer, thyroid cancer, glioblastoma, or glioma;
preferably, the tumor is a hematologic malignancy, such as lymphoma, leukemia; preferably, the lymphoma is Hodgkin's lymphoma or non-Hodgkin's lymphoma; preferably, the non-Hodgkin's lymphoma is one or more of peripheral T-cell lymphoma, angioimmunoblastic T-cell lymphoma, EBV-positive NK/T-cell lymphoma (nasal type), and B-cell non-Hodgkin's lymphoma;
preferably, the infection is selected from a viral infection, a bacterial infection, a fungal infection, and a parasitic infection;
preferably, the subject is a mammal, e.g., a human or a monkey.
